# EUROPEAN PATENT SPECIFICATION

(11) **EP 2 389 589 B1**
(45) Date of publication and mention of the grant of the patent: **12.08.2015**
(21) Application number: 10738941.3
(22) Date of filing: 21.01.2010
(51) Int. Cl.: G01N 33/92

(54) **METHODS OF DIAGNOSING AND TREATING MULTIPLE SCLEROSIS**
VERFAHREN ZUR DIAGNOSE UND BEHANDLUNG VON MULTIPLER SKLEROSE
MÉTHODES DE DIAGNOSTIC ET DE TRAITEMENT DE LA SCLÉROSE EN PLAQUES

(30) Priority: 21.01.2009 US 146160 P
(43) Date of publication of application: 30.11.2011
(73) Proprietor: Brigham and Women's Hospital, Inc., Boston, MA 02115 (US)
(72) Inventor: WEINER, Howard L., Brookline Massachusetts 02445 (US); QUINTANA, Francisco J., Jamaica Plain Massachusetts 02130 (US); FAREZ, Mauricio, Brookline Massachusetts 02446 (US)
(74) Representative: Gowshall, Jonathan Vallance
(86) International application number: PCT/US2010/021598
(87) International publication number: WO 2010/090859

(56) References cited:
- US-A1- 2005 064 516
- US-A1- 2005 089 919
- US-A1- 2007 020 691
- US-A1- 2008 171 394
- US-A1- 2008 268 479
- KANTER JENNIFER L ET AL: "Lipid microarrays identify key mediators of autoimmune brain inflammation.", NATURE MEDICINE JAN 2006 LNKD- PUBMED:16341241, vol. 12, no. 1, January 2006 (2006-01), pages 138-143, XP55036349, ISSN: 1078-8956
- FRANCISCO J QUINTANA ET AL: "Antigen microarrays identify unique serum autoantibody signatures in clinical and pathologic subtypes of multiple sclerosis", PROCEEDINGS OF THE NATIONAL ACADEMY OF SCIENCES, NATIONAL ACADEMY OF SCIENCES, US, vol. 105, no. 48, 12 February 2008 (2008-02-12), pages 18889-18894, XP008147701, ISSN: 0027-8424, DOI: 10.1073/PNAS.0806310105 [retrieved on 2008-11-21]
- W. J. GRIFFITHS ET AL: "Are 15-oxygenated sterols present in the human circulation?", THE JOURNAL OF LIPID RESEARCH, vol. 52, no. 1, 1 January 2011 (2011-01-01), pages 4-5, XP55036334, ISSN: 0022-2275, DOI: 10.1194/jlr.E012088
- I. BJORKHEM ET AL: "High levels of 15-oxygenated steroids in circulation of patients with multiple sclerosis: fact or fiction?", THE JOURNAL OF LIPID RESEARCH, vol. 52, no. 1, 1 January 2011 (2011-01-01), pages 170-174, XP55036335, ISSN: 0022-2275, DOI: 10.1194/jlr.D011072
- INGEMAR BJÖRKHEM ET AL: "Detecting oxysterols in the human circulation", NATURE IMMUNOLOGY, vol. 12, no. 7, 1 January 2011 (2011-01-01), pages 577-577, XP55036336, ISSN: 1529-2908, DOI: 10.1038/ni0711-577a
- MD. OMEDUL ISLAM ET AL: "Generation and Characterization of a Novel Recombinant Antibody Against 15-Ketocholestane Isolated by Phage-Display", INTERNATIONAL JOURNAL OF MOLECULAR SCIENCES, vol. 13, no. 4, 19 April 2012 (2012-04-19) , pages 4937-4948, XP55036338, DOI: 10.3390/ijms13044937
- FRANCISCO J. QUINTANA ET AL: "Lipids and lipid-reactive antibodies as biomarkers for multiple sclerosis", JOURNAL OF NEUROIMMUNOLOGY, 1 May 2012 (2012-05-01), XP55036347, ISSN: 0165-5728, DOI: 10.1016/j.jneuroim.2012.01.002
- MAURICIO F FAREZ ET AL: "Toll-like receptor 2 and poly(ADP-ribose) polymerase 1 promote central nervous system neuroinflammation in progressive EAE", NATURE IMMUNOLOGY, vol. 10, no. 9, 1 September 2009 (2009-09-01), pages 958-964, XP55036350, ISSN: 1529-2908, DOI: 10.1038/ni.1775
- WATERS CORP.: "Waters Quattro Micro GC", , Milford, MA, USA

## Description

### TECHNICAL FIELD

The invention relates to, *inter alia,* methods of using a 15-oxysterol as a biomarker to monitor disease progression in multiple sclerosis (MS).

### BACKGROUND

Multiple Sclerosis (MS) is an inflammatory demyelinating disease of the central nervous system (CNS). MS initially manifests as a relapsing-remitting disease (RRMS), which is followed by the progressive accumulation of neurological disability (secondary progressive MS, SPMS) in the majority of the MS patients. Although several therapies show positive effects on RRMS, they are usually ineffective in SPMS, and no markers are available to monitor the transition to SPMS.

Multiple Sclerosis (MS) is the leading cause of neurological disability in young adults (Noseworthy et al., N Engl J Med 343, 938-952 (2000)). In 85% of the patients MS initially follows a relapsing-remitting course (RRMS) in which acute autoimmune attacks against the central nervous system (CNS) are followed by a complete recovery (Compston and Coles, Lancet 372, 1502-1517 (2008)). The majority of the RRMS patients go on to develop secondary progressive MS (SPMS), characterized by a progressive, irreversible accumulation of neurological disability (Rovaris et al., Lancet Neurol 5, 343-354 (2006)). The progressive and irreversible disability that characterizes SPMS occurs in the absence of new inflammatory lesions, suggesting that other mechanisms might play a role in this stage of MS (Rovaris et al., Lancet Neurol 5, 343-354 (2006)). Indeed, treatments that halt the adaptive inflammatory response show positive effects on the management of RRMS but are usually ineffective in SPMS (Lopez-Diego and Weiner, Nat Rev Drug Discov 7, 909-925 (2008)). Thus, it is important to characterize the processes involved in the transition to SPMS, to identify new therapies for progressive MS and biomarkers to monitor the RRMS to SPMS transition.

Both acute and chronic CNS inflammation and demyelination result in axonal damage (reviewed in Trapp and Nave, Annu Rev Neurosci 31, 247-269 (2008)) which can be detected early in the course of RRMS (Trapp et al., N Engl J Med 338, 278-285 (1998)). Progressive irreversible disability, however, does not characterize these early stages of RRMS probably as a result of compensatory mechanisms operating in the brain (Trapp and Nave, Annu Rev Neurosci 31, 247-269 (2008)). Thus, it has been suggested that the clinical transition to SPMS occurs when the accumulated neuronal loss reaches a threshold that cannot be compensated by the plasticity of the CNS.

A change in the nature of the CNS inflammation has also been linked to the transition to SPMS. When compared with dendritic cells (DC) from healthy controls or RRMS patients, DC from SPMS patients secrete higher levels of IL-12 and IL-18 (Balashov et al., Neurology 55, 192-198 (2000); Balashov et al., Proc Natl Acad Sci U S A 94, 599-603 (1997); Comabella et al.,. J Clin Invest 102, 671-678 (1998); Karni et al., J Neuroimmunol 125, 134-140 (2002)). Moreover, DC from SPMS patients express higher levels of the co-stimulatory molecule CD80, decreased levels of the inhibitory molecule PDL1, and are more efficient in activating Th1 cells (Karni et al., J Immunol 177, 4196-4202 (2006)). Based on these observations and on the limited efficacy of therapies that target the adaptive immune response on SPMS, it has been proposed that while both adaptive and innate immune responses drive the initial RRMS, sustained innate immunity is involved in SPMS (Weiner, J Neurol 255 Suppl 1, 3-11 (2008)).

### SUMMARY

The present inventions is based, at least in part, on the discovery of increased serum levels of the 15-oxysterol 15α-hydroxicholestene (15-HC) in SPMS patients. 15-HC activated microglia, macrophages and astrocytes by a toll like receptor (TLR)-2 dependent signaling pathway that involved the poly(ADP ribose) polymerase-1 (PARP-1). PARP-1 activity in monocytes was up-regulated in RRMS but showed higher levels in SPMS. The inhibition of PARP-1 suppressed axonal loss and progressive disability in experimental SPMS. Thus, 15-HC is a biomarker for monitoring disease progression in MS, and PARP-1 is a new therapeutic target for SPMS.

In one aspect, the invention provides methods for diagnosing multiple sclerosis (MS) in a human subject e.g., in vitro methods of aiding in diagnosis. The methods include evaluating a level of one or more 15-oxysterol, e.g., 15-ketocholestene (15-KE), 15-ketocholestane (15-KA), and/or 15-hydroxycholestene (15-HC), in a serum sample from a human subject by a method comprising subjecting the serum sample to lipid extraction and analysing the sample using tandem quadrupole GC-MS/MS and comparing the level with a reference level. The level of the 15-oxysterol in the sample in comparison to the reference is indicative of whether the subject has MS. In some embodiments, the reference level is a control reference that represents a normal level of the 15-oxysterol, e.g., a level in an unaffected subject, and a level in the sample that is above the control reference indicates that the subject has MS, e.g., RRMS or SPMS. In some embodiments, the reference level is a disease reference that represents a level of the 15-oxysterols, in a subject having MS, e.g., RRMS, or SPMS, and a statistical similarity between the level in the sample and the disease reference indicates that the subject has MS.

In another aspect, the invention features methods for determining whether a human subject has relapsing-remitting MS (RRMS) or secondary progressive MS (SPMS), e.g., in vitro methods of aiding in making the determination. The methods include evaluating a level of one or both of 15-ketocholestene (15-KE) or 15-ketocholestane (15-KA), and also evaluating the presence and/or level of 15-hydroxycholestene (15-HC), in a serum sample from a human subject by a method comprising subjecting the serum sample to lipid extraction and analysing the sample using tandem quadrupole GC-MS/MS and comparing the level of 15-KE, 15-KA, and 15-HC with corresponding references. The level of 15-KE, 15-KA, and 15-HC in the sample as compared to the control reference indicates whether the subject has RRMS or SPMS. In some embodiments, the reference is a control reference that represents a normal level of the 15-KE or 15-KA, and 15-HC in an unaffected subject, and/or a disease reference that represents a level in a subject having RRMS, or SPMS. In some embodiments, the presence of elevated levels of 15-KE or 15-KA, and elevated levels of 15-HC as compared to a control reference, indicates that the subject has SPMS or has an increased risk of developing SPMS, while increased levels of 15-KE, and 15-KA, but not 15-HC, as compared to the control reference indicates that the subject has RRMS, e.g., has not yet progressed to SPMS.

In a further aspect, the invention provides methods for diagnosing SPMS in a human subject, e.g., in vitro methods of aiding in diagnosis. The methods include evaluating a level of 15-hydroxycholestene (15-HC) in a serum sample from a human subject by a method comprising subjecting the serum sample to lipid extraction and analysing the sample using tandem quadrupole GC-MS/MS and comparing the level of 15-HC in the sample with a corresponding reference, wherein the level of 15-HC in the sample as compared to the reference is indicative of whether the subject has SPMS. In some embodiments, the reference is a control reference that represents a normal level of 15-HC in an unaffected subject (in which case, levels in the sample able the control reference level indicate that the subject has, or has a high risk of developing, SPMS), and/or a disease reference that represents a level in a subject having SPMS (in which case levels in the sample that are statistically similar to the disease reference indicate that the subject has, or has a high risk of developing, SPMS). In some embodiments, the presence of elevated 15-HC, e.g., as compared to a control reference, indicates that the subject has SPMS.

Disclosed herein are methods for treating a subject who has SPMS. The methods include selecting a subject on the basis that they have SPMS, and administering to the subject a therapeutically effective amount of a composition comprising a specific inhibitor of poly(ADP ribose) polymerase-1 (PARP-1). Selecting a subject on the basis that they have SPMS can include, e.g., obtaining a sample from a subject; determining a level of 15-hydroxycholestene (15-HC) in the sample; comparing the level of 15-HC in the sample with a corresponding reference representing a level of 15-HC in an unaffected subject, and selecting the subject if the level of 15-HC in the sample is elevated as compared to the reference. Other methods are also known in the art for determining that a subject has SPMS, and selecting subjects on that basis. Also disclosed herein are compositions comprising a specific inhibitor of PARP-1; the use of a specific inhibitor of PARP-1 in the treatment of SPMS; and the use of a specific inhibitor of PARP-1 in the manufacture of a medicament for the treatment of SPMS.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. In case of conflict, the present specification, including definitions, will control.

Other features and advantages of the invention will be apparent from the following detailed description and figures, and from the claims.

### DESCRIPTION OF DRAWINGS

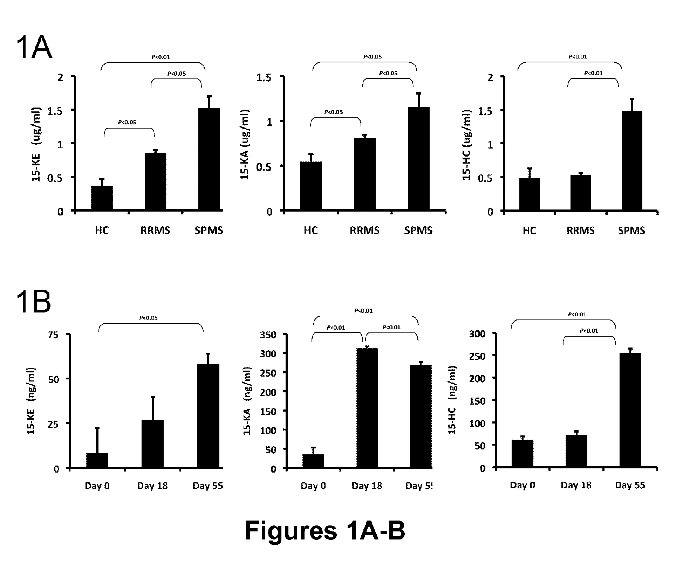

FIGs. 1A and 1B are each sets of three bar graphs showing increased 15-HC serum levels in human and experimental SPMS. 1A, The concentration of 15-oxysterols was measured using GC/MS in healthy controls (n=10), RRMS (n=12) and SPMS patients (n=10). RRMS patients have increased levels of 15-KE (left panel) and 15-KA (middle panel), but not 15-HC (right panel), while SPMS patients have increased levels of all 15-oxysterols as compared to healthy controls and RRMS patients. 1B, The concentration of 15-oxysterols (15-KE (left panel), 15-KA (middle panel), and 15-HC (right panel)) was measured at the peak of the acute attack (day 18) or during the progression phase (day 55) of experimental NOD SPMS. The data is shown as mean ± SEM.

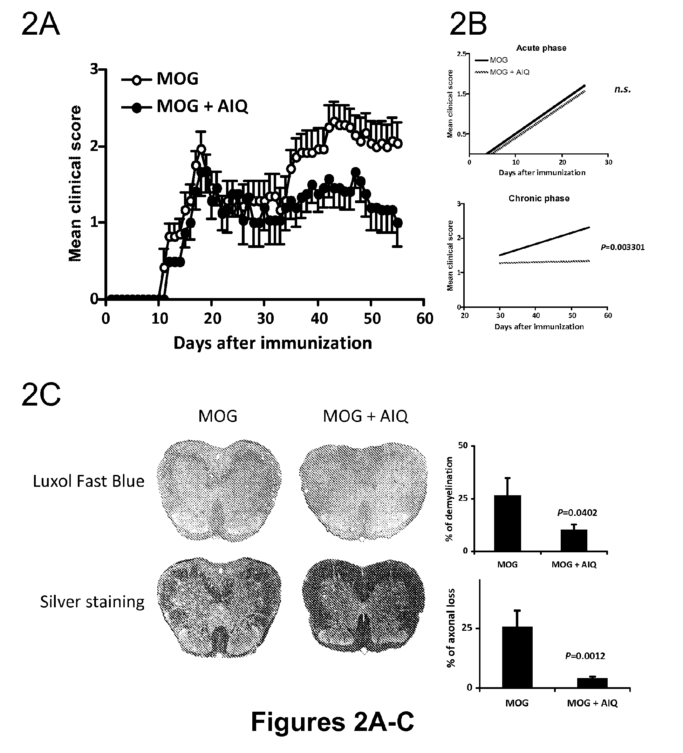

FIGs. 2A-C show that PARP-1 inhibition suppresses axonal loss and progressive disability in experimental SPMS. 2A, a line graph showing the results of AIQ treatment when experimental SPMS was induced in ten-week old NOD mice by subcutaneous immunization with 150 mg of MOG₃₅₋₅₅ in CFA and Pertussis toxin (150ng per mouse) given at the time of immunization and 48 hours later. AIQ (3 mg/Kg body weight) was given daily beginning on day 0, *n*=10 animals per group. A control group was treated with PBS. 2B, a pair of line graphs showing the results of regression analysis of EAE progression (top panel - acute phase, bottom panel-chronic phase). 2C, four images from histopathological analysis of EAE in NOD mice. Representative spinal cord sections from each group taken at day 55, stained with luxol fast blue (top two images) or Bielschowsky's silver staining (bottom two images to analyze demyelination or axonal loss, respectively. Bar graphs at the right of the figure show a significant decrease in percent demyelination (top) and percent axonal loss (bottom) following AIQ treatment.

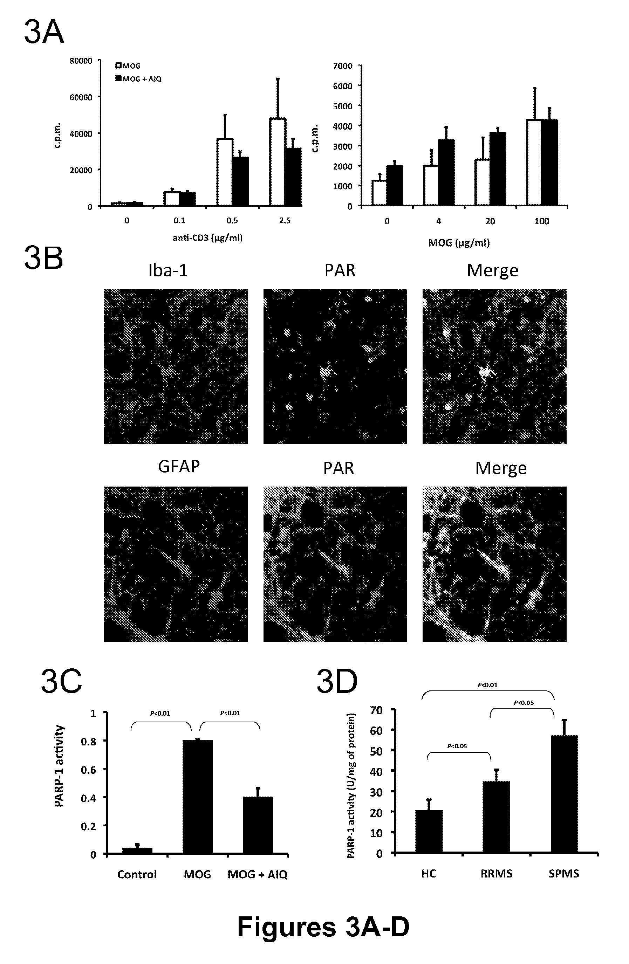

FIGs. 3A-D show that PARP-1 inhibition does not interfere with the adaptive encephalitogenic response in experimental SPMS. 3A, two bar graphs showing the results when splenocytes from vehicle-treated or AIQ treated mice were assayed for their proliferative recall response to MOG₃₅₋₅₅ (right panel) or anti-CD3 (left panel). No statistical differences were observed between the two groups (One-way ANOVA, P>0.05, mean ± SEM). 3B, A set of six photomicrographs of microglial cells and CNS-infiltrating macrophages stained with antibodies to Iba-1 (top left), astrocytes with antibodies to GFAP (bottom left), and PARP-1 activation was followed with antibodies to PAR (middle row); merged images are shown to the right. 3C, a bar graph showing the results of measurement of PARP-1 enzymatic activity in CNS CD11b⁺ cells isolated from AIQ or control-treated mice at day 55 (one way anova, *P*<0.01, mean ± SEM). 3D, a bar graph showing PARP-1 enzymatic activity in peripheral blood monocytes isolated from HC, RRMS and SPMS patients (Mean ± SEM).

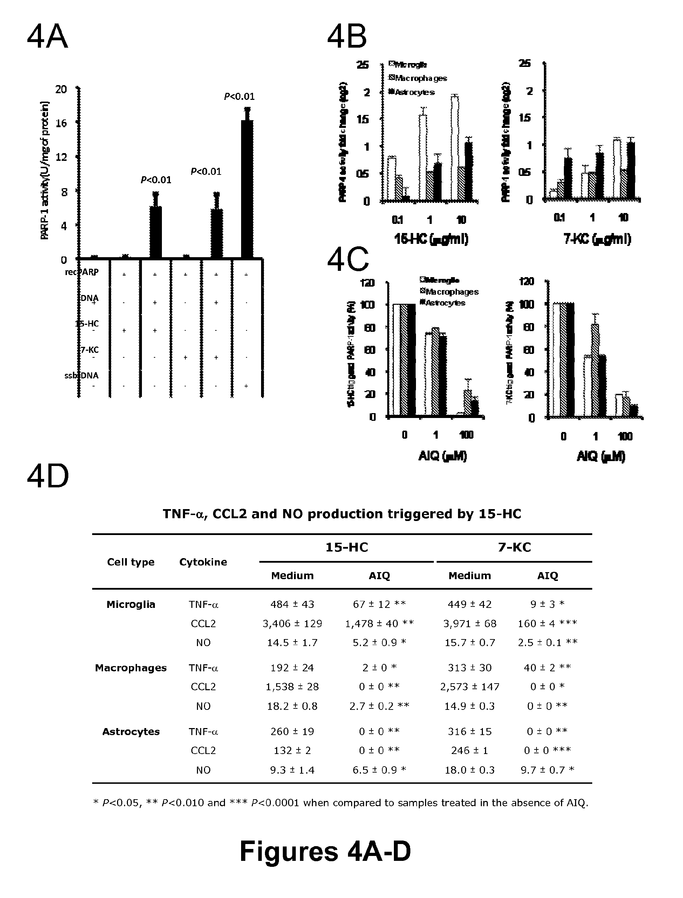

FIGs. 4A-D show the results of activation of microglia, macrophages and astrocytes by 15-HC. 4A, a bar graph showing PARP-1 activity in a cell-free system where recombinant PARP-1 was incubated with DNA which as been pretreated with 15-HC or 7-KC. Non-pretreated DNA was used as a negative control and ssb-DNA as a positive control. Data is shown as Mean ± SEM. 4B, a pair of bar graphs showing PARP-1 activity measured in microglia, macrophages or astrocytes incubated with 15-HC or 7-KC. The data is shown as the fold of change between vehicle and lipid-treated cells (Mean ± SEM). 4C, a pair of bar graphs showing percent inhibition of PARP-1 activity in microglia, macrophages or astrocytes activated with 15-HC or 7-KC (10 mg/ml) in the presence of 5-AIQ. 4D, a table showing levels of TNF-α CCL-2 and NO secretion by 15-HC or 7-KC treated microglia, macrophages or astrocytes (Mean ± SEM).

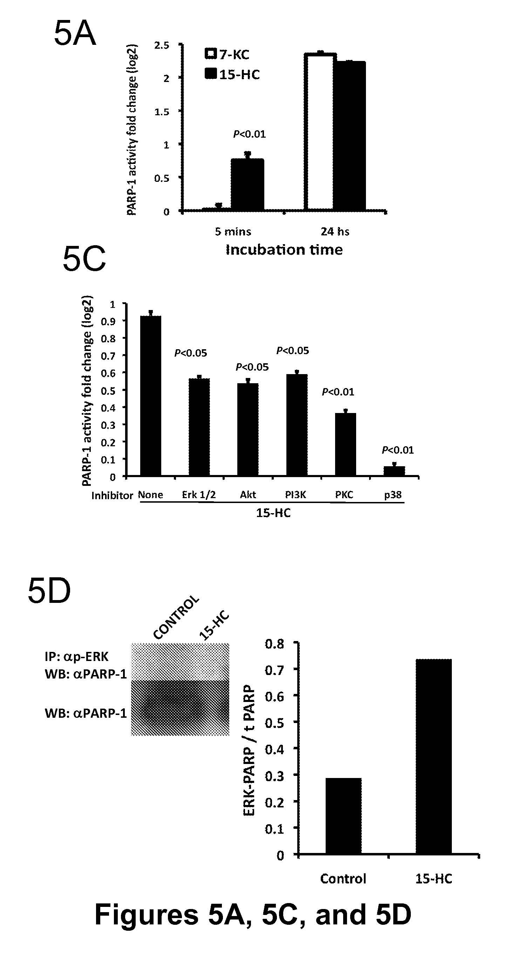

FIGs. 5A-D are bar graphs showing signaling events mediating the activation of PARP-1 by 15-HC, indicating that 15-oxysterols may activate PARP-1 via DNA damage. 5A, a bar graph showing the time course of microglial PARP-1 activation by 15-oxysterols or 7-KC. PARP-1 activity is shown as Mean ± SEM. 5B, a set of eight bar graphs showing signaling pathways activated by 15-HC. Microglial cells were stimulated with 15-HC and lysed at 1,5, and 15 minutes. Signal transduction arrays were made with the lysates and probed with phospho-specific antibodies. 15a-HC increases the phosphorylation of TAK1 (top right), IRAK (top left), Akt (second row, left), p38 MAPK (second row, right), Pyk2 (third row, left), PKC (third row, right), PI3K (bottom row, left), and Erkl/2 (bottom row, right). (Mean ± SEM) 5C, a bar graph showing activation of microglial PARP-1 by 15-HC in the presence of kinase inhibitors (Mean ± SEM). 5D, an immunoblot and a bar graph showing the results of co-immunoprecipitaiton of ERK and PARP-1. Microglial cells were treated with 15-HC, lysed, immunoprecipitated with antibodies to phosphorylated Erk and then the pulled down material was probed with antibodies to PARP-1.

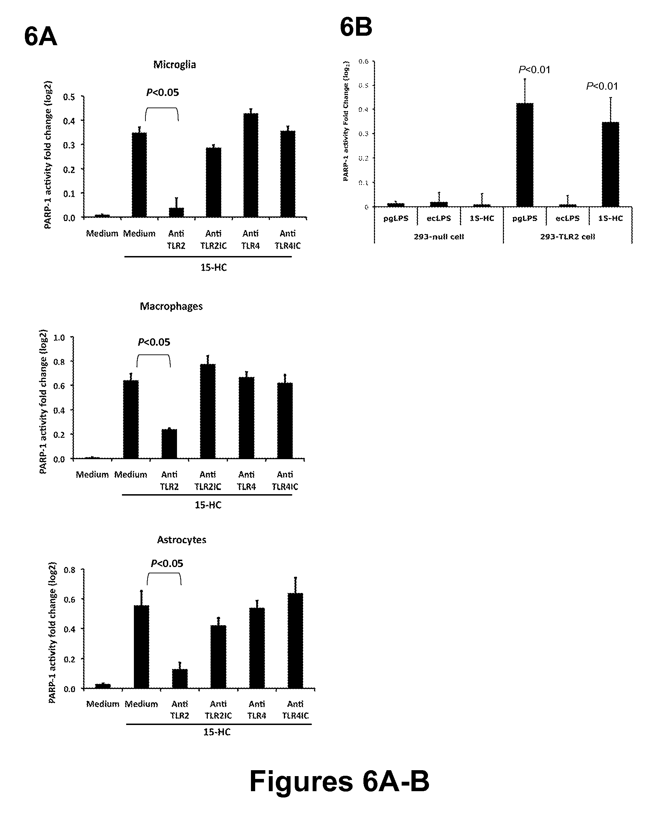

FIGs. 6A-B are bar graphs showing that PARP-1 activation by 15-HC is mediated by TLR2 A. TLR2 blocking antibody abrogates PARP-1 activation by 15a-HC. 6A, a set of three bar graphs showing PARP-1 activity in microglia, macrophages or astrocytes activated with 15-HC in the presence of blocking antibodies to TLR2 or TLR4, or isotype controls (Mean ± SEM). 6B, a bar graph showing PARP-1 activity in HEK-293 cells transfected or not with a plasmid coding for TLR2 and activated with 15-HC (Mean ± SEM).

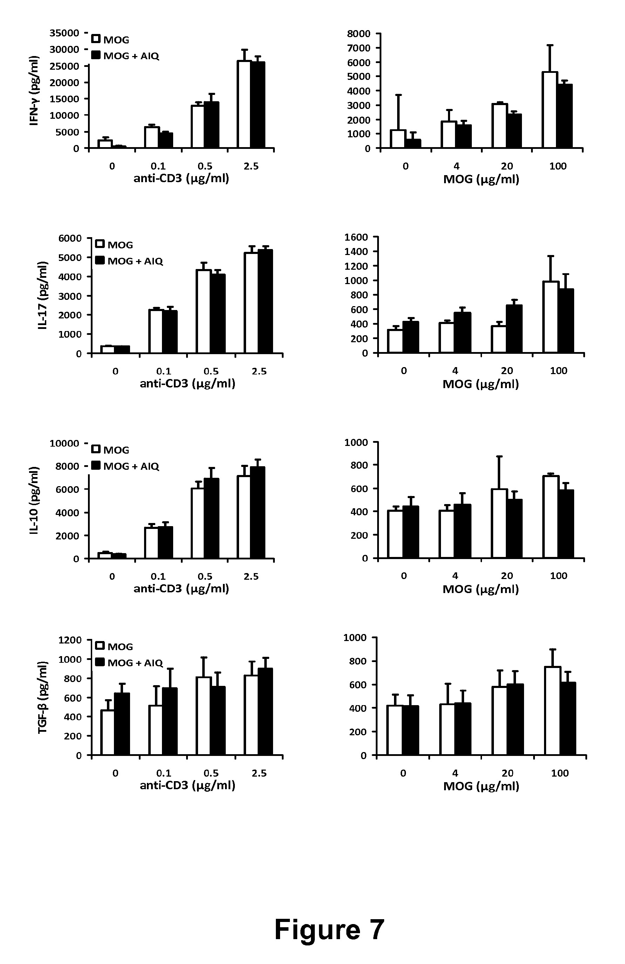

FIG. 7 is a set of eight bar graphs showing that the adaptive encephalitogenic immune response is not affected by the inhibition of PARP-1. Splenocytes prepared from control or AIQ-treated mice were activated in vitro with MOG35-55 (right column) or antibodies to CD3 (left column) and the cytokines levels were tested on the supernatant at 48 hours. Top row, IFN-gamma. Second row, IL-17. Third row, IL-10. Bottom row, TGF-beta.

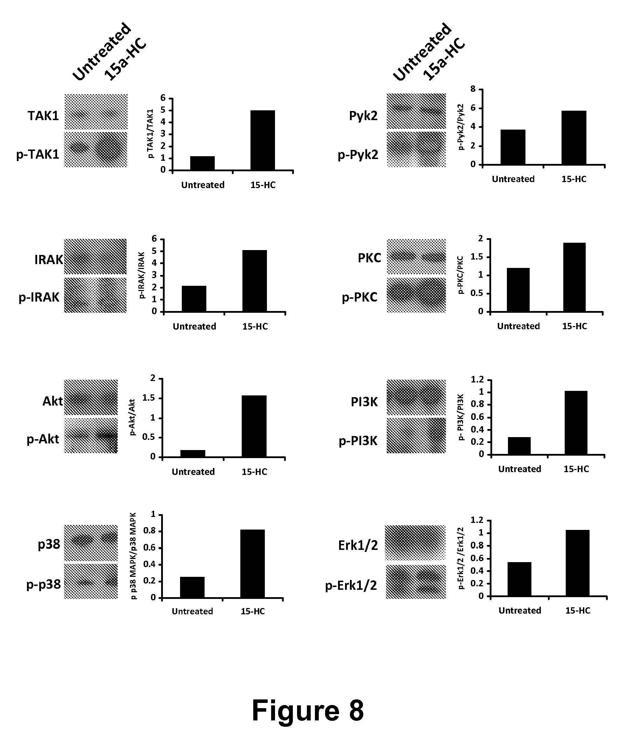

FIG. 8 is a set of eight pairs of bolts and bar graphs showing blockade of 15-HC triggered signaling pathways with blocking antibodies to TLR2. Microglial cells were incubated with 10mg/ml of TLR2 blocking antibody or isotype control 45 minutes prior the addition of 15-HC to the medium and signal transduction events were analyzed by western blot.

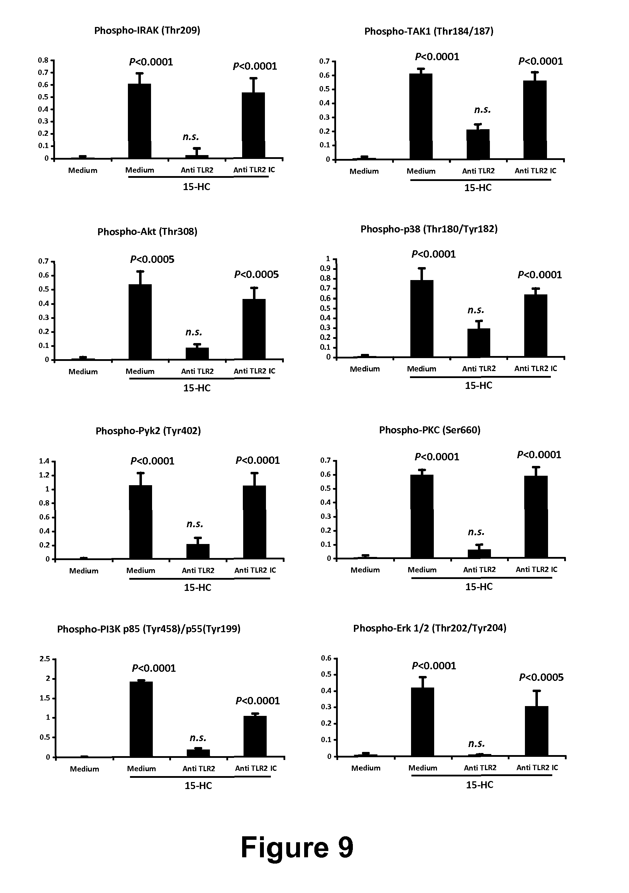

FIG. 9 is a set of eight bar graphs showing signaling pathways activated by 15-HC, validated by Western blot. Cell lysates prepared in similar conditions to those used for the construction of RPA were analyzed by Western blot to detect total and phosphorylated protein levels of the targets of interest identified with RPA. The ratio of phosphor-protein/total protein is shown.

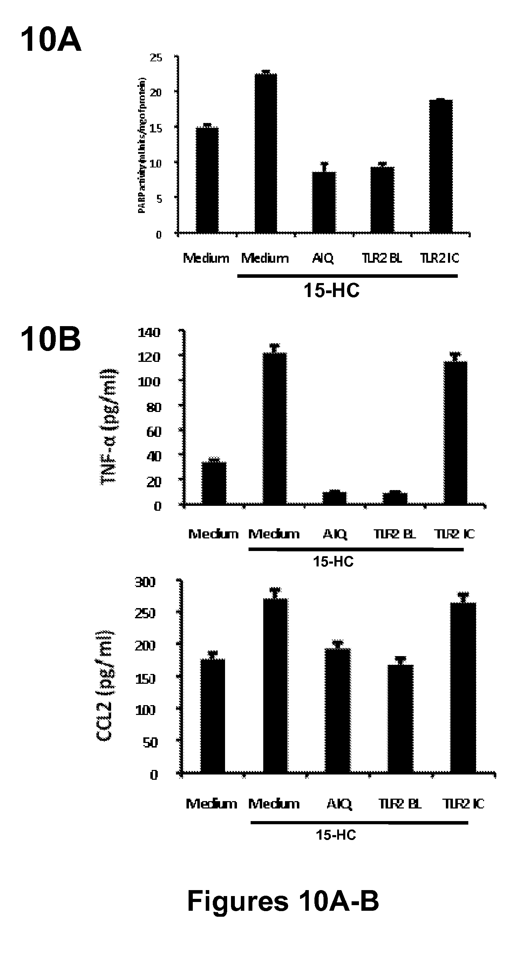

FIGs 10A and 10B are bar graphs showing TLR2 mediated activation of PARP-1 by 15-HC. PARP-1 enzymatic activity (10A) and TNFalpha and CCL2 (10B) of PBMCs incubated with 15-HC alone, or in combination with AIQ, blocking antibodies to TLR2 or IC control.
FIG.s 11A-B are show the results of FACS-based detection of PARP-1 activity. 11A. a set of five histograms showing PAR staining (as detected by FACS) of PBMCs incubated with 15-HC alone, or in combination with AIQ, blocking antibodies to TLR2 (TLR2 BL), or IC control (TLR2 IC). 11B, a line graph showing the correlation of PAR staining as detected by FACS with PARP-1 enzymatic activity detected on PBMCs activated with different concentration of 15-HC.

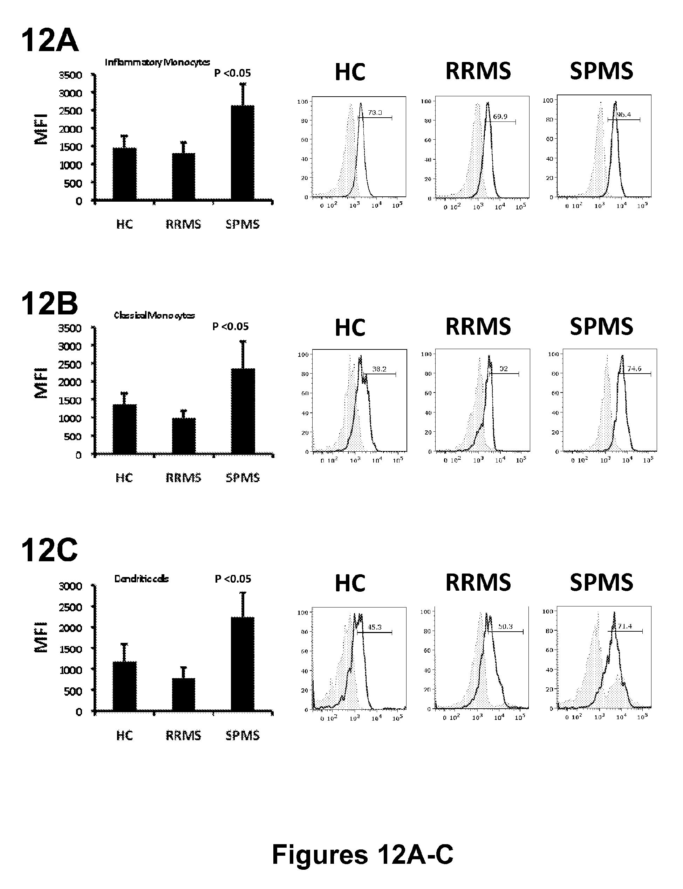

FIGs. 12A-C are each bar graphs paired with three histograms showing increased PARP-1 activity in cells of the innate immune system of SPMS patients. Mean PARP-1 activity as determined by FACS in inflammatory monocytes (12A), classical monocytes (12B) and dendritic cells (12C) of healthy controls (HC), RRMS and SPMS patients. The data are shown as mean fluorescence intensity (MFI) ± SD of 5 samples/group. Representative FACS plots of PAR staining are also shown.
FIG.s 13A-B are each bar graphs paired with three histograms showing increased PARP-1 activity in cells of the adaptive immune system of RRMS and SPMS patients. Mean PARP-1 activity as determined by FACS in CD8+ T cells (13A), B cells (13B) and CD4+T cells (13C) of healthy controls (HC), RRMS and SPMS patients. The data are shown as mean fluorescence intensity (MFI) ± SD of 5 samples/group. Representative FACS plots of PAR staining are also shown.

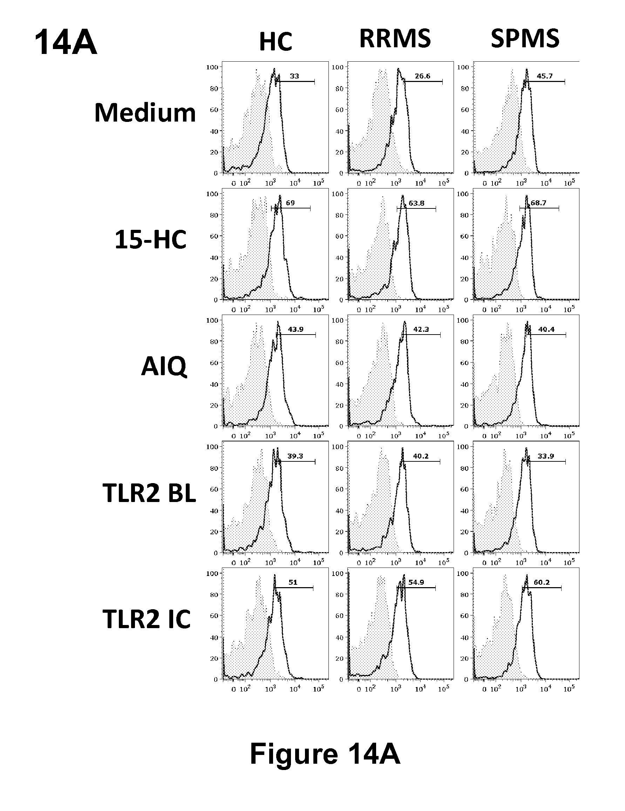

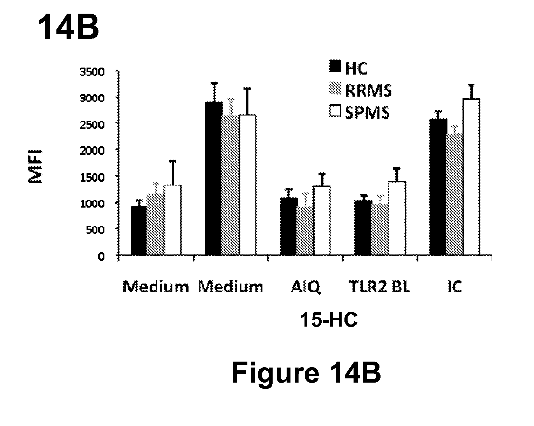

FIGs. 14A and 14B show that 15-HC induces similar levels of PARP-1 activation via TLR2 in HC, RRMS and SPMS. 14A is a set of fifteen histograms showing PAR staining of PBMCs taken from HC (left column), RRMS (center column) or SPMS (right column) patients and unstimulated (top row) or activated with 15-HC alone (second row), or in combination with AIQ (third row), blocking antibodies to TLR2 (fourth row) or IC control (bottom row). 14B is a bar graph showing the mean fluorescence intensity (MFI) ± SD of 5 samples/group from the histograms in 14A.

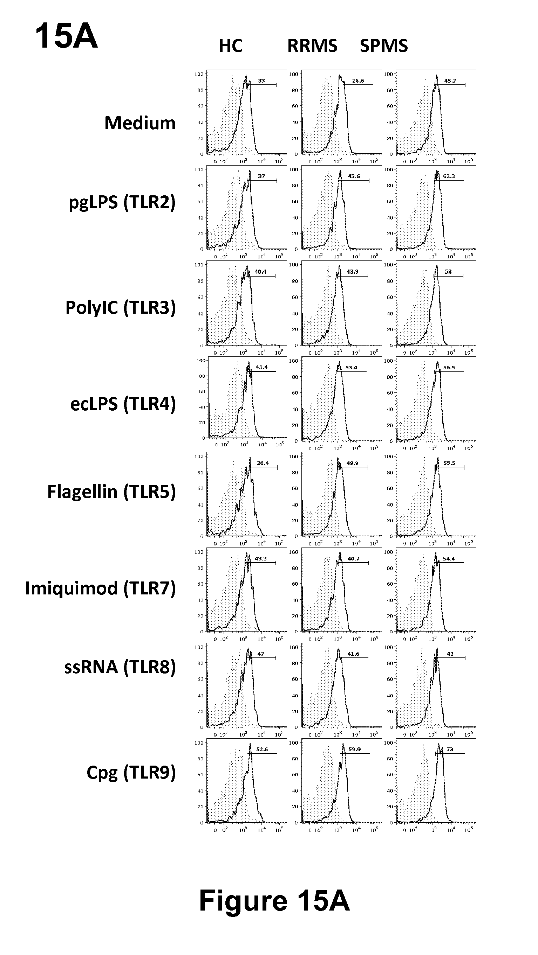

FIGs. 15A-B show that similar levels of PARP-1 activation are triggered by TLR agonists in HC, RRMS and SPMS. 15A is a set of 24 histograms showing PAR staining of PBMCs taken from HC (left column), RRMS (center column) or SPMS (right column) patients and unstimulated (top row) or activated with pgLPS (second row), polyIC (third row), ecLPS (fourth row), flagellin (row 5), imiquimod (row 6), ssRNA (row 7)or CpG (row 8). 15B is a bar graph showing the mean fluorescence intensity (MFI) ± SD of 5 samples/group from the histograms in 14A.

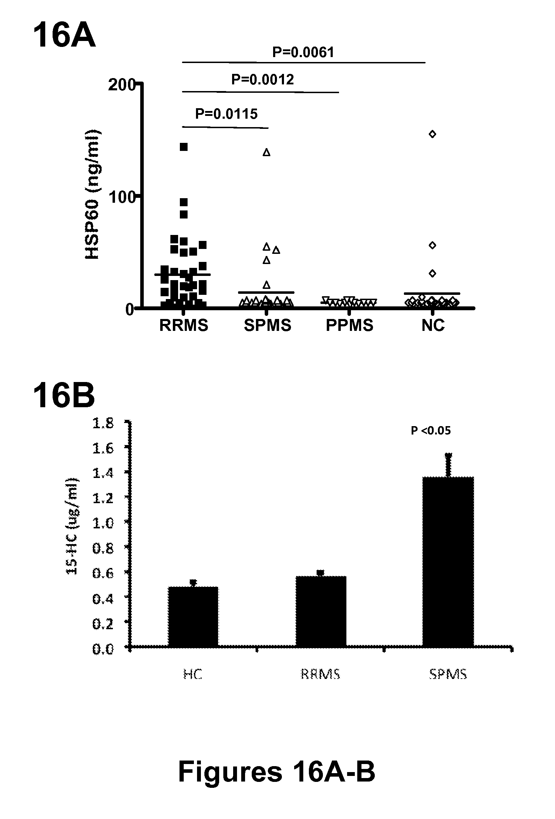

FIGs. 16A-B are a dot graph and a bar graph, respectively, showing increased levels of the TLR2 agonists HSP60 in the RRMS patients (16A), and 15-HC in the SPMS patients (16B). Levels of HSP60 (16A) and 15-HC (16B) were determined in serum samples taken from HC, RRMS and SPMS patients.

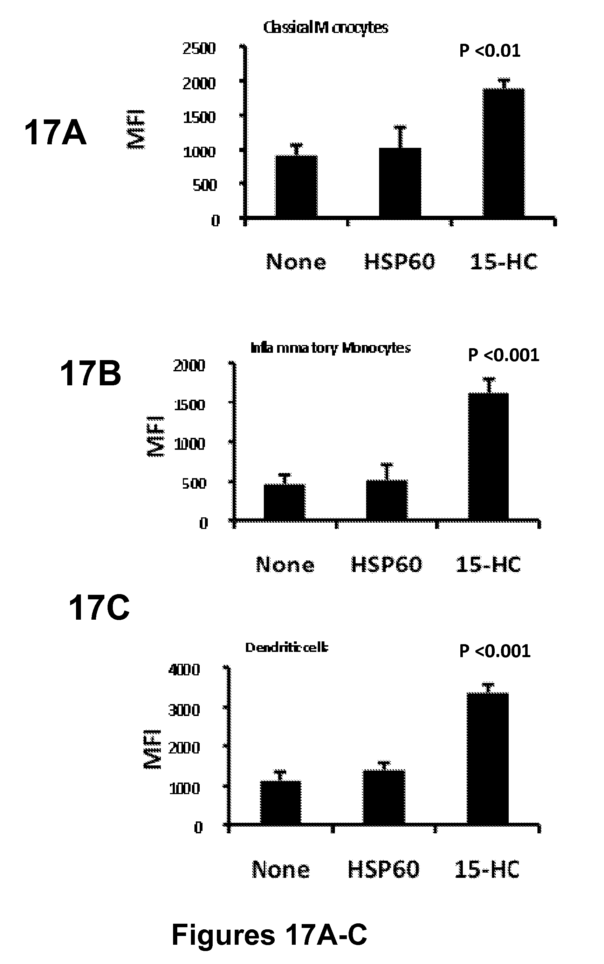

FIGs. 17A-C are bar graphs showing that 15-HC, but not HSP60, activates PARP-1 in cells of the innate immune system. Mean PARP-1 activity as determined by FACS in classical monocytes (17A), inflammatory monocytes (17B) and dendritic cells (17C) of healthy controls (HC) treated with activated with HSP60, 15-HC or left untreated. The data are shown as mean fluorescence intensity (MFI) ± SD of 5 samples/group.

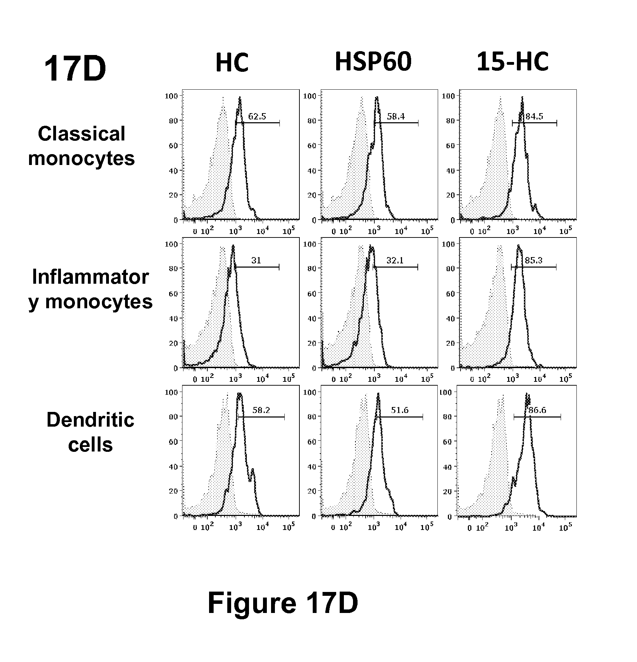

FIG. 17D is a set of nine representative FACS plots of PAR staining from the experiments shown in Figs. 17A-C.

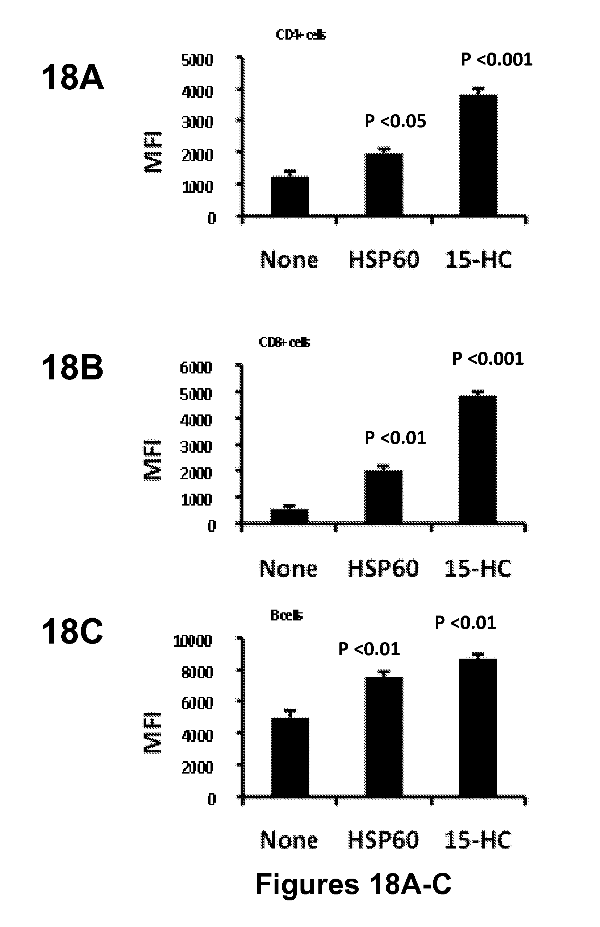

FIGs. 18A-C are bar graphs showing that both 15-HC and HSP60 activate PARP-1 in cells of the adaptive immune system. Mean PARP-1 activity as determined by FACS in CD4+ T cells (18A), CD8+ T cells (18B) and B cells (18C) of healthy controls (HC) treated with activated with HSP60, 15-HC or left untreated. The data are shown as mean fluorescence intensity (MFI) ± SD of 5 samples/group.

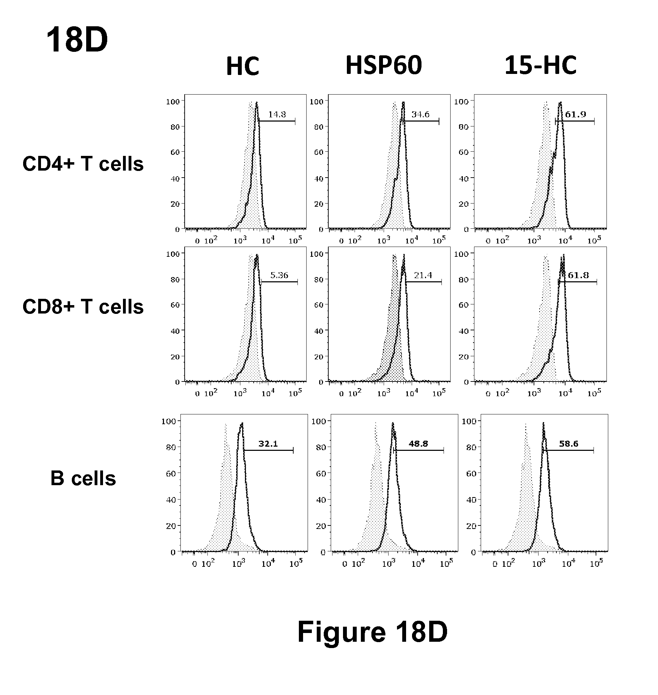

FIG. 18D is a set of nine representative FACS plots of PAR staining from the experiments shown in Figs. 18A-C.

### DETAILED DESCRIPTION

In the majority of patients, MS eventually adopts a progressive course that shows a limited response to therapy (Rovaris et al., Lancet Neurol 5, 343-354 (2006); Lopez-Diego and Weiner, Nat Rev Drug Discov 7, 909-925 (2008)). Thus, the investigation of processes that contribute to the progressive phase of MS and the identification of biomarkers to monitor disease progression is a priority in MS research.

Using antigen microarrays (Quintana et al., Proc Natl Acad Sci U S A 101 Suppl 2, 14615-14621 (2004); Robinson et al., Nat Biotechnol 21, 1033-1039 (2003)) the present inventors detected increased levels of antibodies to 15-oxysterols, oxidized derivatives of cholesterol, in the serum of MS patients (Quintana et al., Proc Natl Acad Sci U S A 105, 18889-18894 (2008)). 7-ketocholesterol (7-KC), an oxidized derivative of cholesterol that is not a 15-oxysterol, has been shown to activate microglia through a mechanism mediated by poly(ADP ribose) polymerase-1 (PARP-1) that results in neuronal damage in vitro (Diestel et al., J Exp Med 198, 1729-1740 (2003)). Based on the importance of neuronal death for the pathogenesis of progressive MS the role of 15-oxysterols in MS and EAE was investigated. There were increased serum levels of the 15-oxysterol 15a-hydroxicholestene (15-HC) in progressive MS and EAE. As demonstrated herein, in vitro, 15-HC activates macrophages, microglia and astrocytes via a signaling pathway that includes toll like receptor-2 (TLR2) and PARP-1. The inhibition of PARP-1 suppressed axonal loss and disability in a mouse model of SPMS. Thus, 15-HC is a potential biomarker to follow disease progression in MS, and the TLR2 / PARP-1 axis is a therapeutic target for SPMS.

### Methods of Treating MS/SPMS

Multiple Sclerosis (MS) is typically characterized clinically by recurrent or chronically progressive neurologic dysfunction, caused by lesions in the CNS. Pathologically, the lesions include multiple areas of demyelination affecting the brain, optic nerves, and spinal cord. The underlying etiology is uncertain, but MS is widely believed to be at least partly an autoimmune or immune-mediated disease.

Secondary Progressive Multiple Sclerosis (SPMS), one of four internationally recognized forms of Multiple Sclerosis (the others being Relapsing/Remitting Multiple Sclerosis, Primary Progressive Multiple Sclerosis and Progressive Relapsing Multiple Sclerosis), is characterized by a steady progression of clinical neurological damage with or without superimposed relapses and minor remissions and plateaus. People who develop SPMS will generally have previously suffered a period of Relapsing/Remitting Multiple Sclerosis (RRMS), which may have lasted from two to forty years or more. Occasionally the subject will have some relapses and remissions after the development of SPMS, but these tend to become less frequent over time.

A diagnosis of MS can be made on the basis of the presence of CNS lesions disseminated in space and time, and the elimination of alternative diagnoses (Problems of experimental trials of therapy in multiple sclerosis: Report by the panel on the evaluation of experimental trials of therapy in multiple sclerosis. Ann N Y Acad Sci. 122: 1965; 552-568). Alternatively, a diagnosis can be made based on the presence of clinical signs and symptoms including heat sensitivity, internuclear ophthalmoplegia, optic neuritis, and Lhermitte symptom (see, e.g., McDonald et al., Recommended Diagnostic Criteria for Multiple Sclerosis: Guidelines From the International Panel on the Diagnosis of Multiple Sclerosis. Ann. Neurol. 2001; 50:121; and Polman et al., Diagnostic Criteria for Multiple Sclerosis: 2005 Revisions to the "McDonald Criteria." Ann Neurol 2005;58:840-846).

Methods of quantifying disability in MS include the Kurtzke Expanded Disability Status Scale (EDSS); MRI scanning; The Scripps Neurologic Rating Scale (SNRS); The Krupp Fatigue Severity Scale (FSS); The Incapacity Status Scale (ISS); The Functional Independence Measure (FIM); The Ambulation Index (AI); The Cambridge Multiple Sclerosis Basic Score (CAMBS); The Functional Assessment of Multiple Sclerosis (FAMS); Profile of Mood States (POMS); and the Sickness Impact Profile (SIP).

Further information about MS, and SPMS, be found in the art, e.g., in Spinal Cord Medicine, Principles and Practice, Lin et al., Eds., (Demos Medical Publishing, Inc., 2003), e.g., Section V, Chapter 32, "Multiple Sclerosis".

The methods described herein include methods for the treatment of SPMS using specific inhibitors of PARP-1. The methods include identifying a subject having SPMS, and administering a therapeutically effective amount of a specific inhibitor of PARP-1, e.g., a therapeutic composition comprising the specific inhibitor of PARP-1, to a subject who has SPMS.

In one aspect, the methods include screening for subjects for SPMS, e.g., by screening for one or more indicators of SPMS. Methods for identifying subjects with SPMS are known in the art, and can also include identifying subjects by detecting elevated levels of 15-HC as described herein. In some embodiments, SPMS is identified by progression of disability in a subject with MS to an EDSS of 3.5 or greater, usually in motor/cerebellar functions.

In some embodiments, the specific inhibitor of PARP-1 is administered in combination with a standard treatment for MS, e.g., administration of corticosteroid therapy, interferon beta-1b, Glatiramer, mitoxantrone, cannabis, or a combination thereof. In some embodiments, the specific inhibitor of PARP-1 is administered in combination with a treatment for one or more symptoms of MS, e.g., depression and fatigue, bladder dysfunction, spasticity, pain, ataxia, and intention tremor; such treatments include pharmacological agents, exercise, and appropriate orthotics. Additional information on the diagnosis and treatment of MS can be found at the National MS Society website, on the world wide web at nationalmssociety.org, the contents of which are incorporated by reference herein.

As used in this context, to "treat" means to ameliorate at least one symptom of the disorder associated with SPMS. A treatment can result in a reduction in one or more symptoms of MS, e.g., depression and fatigue, bladder dysfunction, spasticity, pain, ataxia, and intention tremor. A therapeutically effective amount can be an amount sufficient to prevent the onset of an acute episode or to shorten the duration of an acute episode, or to decrease the severity of one or more symptoms, e.g., heat sensitivity, internuclears ophthalmoplegia, optic neuritis, and Lhermitte symptom. In some embodiments, a therapeutically effective amount is an amount sufficient to prevent the appearance of, delay or prevent the growth (i.e., increase in size) of, or promote the healing of a demyelinated lesion in one or more of the brain, optic nerves, and spinal cord of the subject, e.g., as demonstrated on MRI.

Dosage, toxicity and therapeutic efficacy of the compounds can be determined, e.g., by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

An "effective amount" is an amount sufficient to effect beneficial or desired results. For example, a therapeutic amount is one that achieves the desired therapeutic effect. This amount can be the same or different from a prophylactically effective amount, which is an amount necessary to prevent onset of disease or disease symptoms. An effective amount can be administered in one or more administrations, applications or dosages. A therapeutically effective amount of a composition depends on the composition selected. The compositions can be administered one from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of the compositions described herein can include a single treatment or a series of treatments.

### Methods of Diagnosis

Disclosed herein are methods for diagnosing MS, or for identifying subjects who have SPMS or RRMS. The methods include obtaining a sample comprising serum from a subject, and evaluating the presence and/or level of one or more 15-oxysterol, e.g., 15-ketocholestene (15-KE), 15-ketocholestane (15-KA) and 15-hydroxycholestene (15-HC), in the sample, and comparing the presence and/or level with one or more references, e.g., a control reference that represents a normal level of the 15-oxysterol, e.g., a level in an unaffected subject, and/or a disease reference that represents a level of the 15-oxysterols, in a subject having MS, RRMS, or SPMS. Suitable reference values can include those shown in Fig. 1A, e.g., above about 0.3, 0.35, 0.4, 0.45, 0.5, 0.55, 0.6, or 0.7 ug/ml. The presence and/or level of a 15-oxysterol can be evaluated using methods known in the art, e.g., using gas chromatography/mass spectrometry (GC/MS), dilution mass spectrometry, and/or gas-liquid chromatography.

In some embodiments, the presence and/or level of 15-oxysterol is comparable to the presence and/or level of the 15-oxysterol in the disease reference, and the subject has one or more symptoms associated with MS, then the subject has MS. In some embodiments, the subject has no overt signs or symptoms of MS, RRMS, or SPMS, but the presence and/or level of one or more of the 15-oxysterol evaluated is comparable to the presence and/or level of the protein(s) in the disease reference, then the subject has an increased risk of developing MS, RRMS, or SPMS. The sample is serum. In some embodiments, once it has been determined that a person has MS, RRMS, or SPMS, or has an increased risk of developing RRMS or SPMS, then a treatment, e.g., as known in the art or as described herein, can be administered.

In some embodiments, the methods include evaluating the presence and/or level of one or both of 15-ketocholestene (15-KE) or 15-ketocholestane (15-KA), and also evaluating the presence and/or level of 15-hydroxycholestene (15-HC), in the sample, and comparing the presence and/or level of the 15-KE, 15-KA, and 15-HC with corresponding references, e.g., a control reference that represents a normal level of the 15-KE, 15-KA, and 15-HC in an unaffected subject, and/or a disease reference that represents a level in a subject having RRMS, or SPMS. In some embodiments, a differential diagnosis of RRMS or SPMS is made; the presence of elevated levels of 15-KE, 15-KA, and 15-HC as compared to a reference indicates that the subject has SPMS or has an increased risk of developing SPMS, while increased levels of 15-KE, and 15-KA, but not 15-HC, as compared to the reference indicates that the subject has RRMS, e.g., has not yet progressed to SPMS.

In some embodiments, the methods include evaluating the presence and/or level of 15-hydroxycholestene (15-HC), in the sample, and comparing the presence and/or level of 15-HC with corresponding references, e.g., a control reference that represents a normal level of 15-HC in an unaffected subject, and/or a disease reference that represents a level in a subject having SPMS. The presence of elevated 15-HC indicates that the subject has SPMS.

### Pharmaceutical Compositions Comprising PARP-1 inhibitors

The methods described herein include the manufacture and use of pharmaceutical compositions, which include specific inhibitors of PARP-1 as active ingredients. Also included are the pharmaceutical compositions themselves.

A number of PARP-1 inhibitors are known in the art, including 5-chloro-2-[3-(4-phenyl-3, 6-dihydro-1(2H)-pyridinyl)propyl]-4(3H)-quinazolinone (FR247304) (Iwashita et al., JPET 2004; 310:425-436); PJ34 (Faro et al., Ann Thorac Surg. 2002 Feb;73(2):575-81); 2-[(R)-2-methylpyrrolidin-2-yl]-1H-benzimidazole-4-carboxamide (ABT-888) (Penning et al., J Med Chem. 2009 Jan 22;52(2):514-23); Quinoline-8-carboxamides (Lord et al., J Med Chem. 2008 Dec 31. [Epub ahead of print]); 5-Aminoisoquinolin-1-one (5AIQ) (Cai et al., Pathol Oncol Res. 2008 Nov 7. [Epub ahead of print]); nicotinamide; 3-aminobenzamide (3-AB)(Czapski et al., Neurosci Lett. 2004 Feb 6;356(1):45-8); benzamide; 3,4-dihydro-5-methoxyisoquinolin-1(2H)-one (PD 128763) or 8-hydroxy-2-methylquinazolin-4(3H)-one (NU1025) (Boulton et al., Br J Cancer. 1995 Oct;72(4):849-56); 2-methylbenzimidazole-4-carboxamide (NU1064) (Bowman et al., Br J Cancer. 1998 Nov;78(10): 1269-77); 3-aminobenzamide; 4-amino-1,8-naphthalimide (Sharma et al., Life Sci. 2008 Mar 12;82(11-12):570-6. Epub 2007 Dec 26.; 1,5-isoquinolinediol (Dalmau et al., Exp Cell Res. 1996 Oct 10;228(1):14-8); 6(5H)-phenanthridinone (Weltin et al., Oncol Res. 1994;6(9):399-403); adenosine substituted 2,3-dihydro-1H-isoindol-1-ones; AG14361 (Veuger et al., Cancer Res. 2003 Sep 15;63(18):6008-15); AG014699 (Thomas et al., Mol Cancer Ther. 2007 Mar;6(3):945-56); 2-(4-chlorophenyl)-5-quinoxalinecarboxamide (FR261529)(Iwashita et al., J Pharmacol Exp Ther. 2004 Sep;310(3):1114-24. Epub 2004 Apr 27); isoindolinone derivative INO-1001 (Shimoda et al., Am J Physiol Lung Cell Mol Physiol. 2003 Jul;285(1):L240-9. Epub 2003 Mar 7); 4-hydroxyquinazoline (4-HQN) (J Pharmacol Exp Ther. 2004 Jul;310(1):247-55. Epub 2004 Mar 3); 2-[3-[4-(4-chlorophenyl)-1-piperazinyl]propyl]-4-3(4)-quinazolinone (FR255595) (Iwashita et al., J Pharmacol Exp Ther. 2004 Jun;309(3):1067-78. Epub 2004 Feb 25); isoqoinolinone derivatives, e.g., 5-benzoyloxyisoquinolin-1(2 H)-one (Pellicciari et al., ChemMedChem. 2008 Jun;3(6):914-23); 1,5-dihydroxyisoquinoline (DHIQ) (Czapski et al., Neurosci Lett. 2004 Feb 6;356(1):45-8); 3,4-dihydro-5 [4-(1-piperidinyl)(butoxy)-1(2H)-isoquinolone; CEP-6800 (Miknyocczki et al., Mol Cancer Ther. 2003 Apr;2(4):371-82); 4-amino-1,8-napthalimide (Kabra et al., Brain Res Bull. 2004 Feb 1;62(5):425-33); GPI-15427 (Tentori et al., Clin Cancer Res. 2003 Nov 1;9(14):5370-9); 3,4-dihydro-5-[4-(1-piperidinyl)butoxy]-1(2H)-isoquinolinone (DPQ) (Takahashi et al., Brain Res. 1999 May 22;829(1-2):46-54); BS- 201; 4-[3-(4-Cyclopropanecarbonylpiperazine-1-carbonyl)-4-fluorobenzyl]-2H-phthalazin-1-one (KU-0059436, AZD2281)(Menear et al., J Med Chem. 2008 Oct 23;51(20):6581-91. Epub 2008 Sep 19); 5-iodo-6-aminobenzopyrone (INH2BP) (Endres et al., Eur J Pharmacol. 1998 Jun 26;351(3):377-82); and TIQ-A (Oumouna et al., J Immunol. 2006 Nov 1;177(9):6489-96).

Pharmaceutical compositions typically include a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with the intended route of administration. Examples of routes of administration include parenteral, e.g., intravenous, intradermal, subcutaneous, oral (e.g., inhalation), transdermal (topical), transmucosal, and rectal administration. Methods of formulating suitable pharmaceutical compositions are known in the art, see, e.g., the books in the series Drugs and the Pharmaceutical Sciences: a Series of Textbooks and Monographs (Dekker, NY); and Allen et al., Ansel's Pharmaceutical Dosage Forms and Drug Delivery Systems, Lippincott Williams & Wilkins; 8th edition (2004). The pharmaceutical compositions can be included in a container, pack, or dispenser together with instructions for administration.

### Methods of Screening

Also disclosed herein are methods for screening test compounds, e.g., polypeptides, polynucleotides, inorganic or organic large or small molecule test compounds, to identify agents useful in the treatment of SPMS.

As used herein, "small molecules" refers to small organic or inorganic molecules of molecular weight below about 3,000 Daltons. In general, small molecules useful for the invention have a molecular weight of less than 3,000 Daltons (Da). The small molecules can be, e.g., from at least about 100 Da to about 3,000 Da (e.g., between about 100 to about 3,000 Da, about 100 to about 2500 Da, about 100 to about 2,000 Da, about 100 to about 1,750 Da, about 100 to about 1,500 Da, about 100 to about 1,250 Da, about 100 to about 1,000 Da, about 100 to about 750 Da, about 100 to about 500 Da, about 200 to about 1500, about 500 to about 1000, about 300 to about 1000 Da, or about 100 to about 250 Da).

The test compounds can be, e.g., natural products or members of a combinatorial chemistry library. A set of diverse molecules should be used to cover a variety of functions such as charge, aromaticity, hydrogen bonding, flexibility, size, length of side chain, hydrophobicity, and rigidity. Combinatorial techniques suitable for synthesizing small molecules are known in the art, e.g., as exemplified by Obrecht and Villalgordo, Solid-Supported Combinatorial and Parallel Synthesis of Small-Molecular-Weight Compound Libraries, Pergamon-Elsevier Science Limited (1998), and include those such as the "split and pool" or "parallel" synthesis techniques, solid-phase and solution-phase techniques, and encoding techniques (see, for example, Czarnik, Curr. Opin. Chem. Bio. 1:60-6 (1997)). In addition, a number of small molecule libraries are commercially available. A number of suitable small molecule test compounds are listed in U.S. Patent No. 6,503,713, incorporated herein by reference in its entirety

Libraries screened using the methods of the present invention can comprise a variety of types of test compounds. A given library can comprise a set of structurally related or unrelated test compounds. In some embodiments, the test compounds are peptide or peptidomimetic molecules. In some embodiments, the test compounds are nucleic acids.

In some embodiments, the test compounds and libraries thereof can be obtained by systematically altering the structure of a first test compound, e.g., a first test compound that is structurally similar to a known natural binding partner of the target polypeptide, or a first small molecule identified as capable of binding the target polypeptide, e.g., using methods known in the art or the methods described herein, and correlating that structure to a resulting biological activity, e.g., a structure-activity relationship study. As one of skill in the art will appreciate, there are a variety of standard methods for creating such a structure-activity relationship. Thus, in some instances, the work may be largely empirical, and in others, the three-dimensional structure of an endogenous polypeptide or portion thereof can be used as a starting point for the rational design of a small molecule compound or compounds. For example, in one embodiment, a general library of small molecules is screened, e.g., using the methods described herein.

In some embodiments, a test compound is applied to a test sample, e.g., a cell or living tissue or organ, and one or more effects of the test compound is evaluated. In a cultured or primary cell for example, the ability of the test compound to inhibit PARP-1 is evaluated.

In some embodiments, the test sample is, or is derived from (e.g., a sample taken from) an *in vivo* model of a disorder as described herein. For example, an animal model, e.g., a rodent such as a rat, can be used.

Methods for evaluating each of these effects are known in the art. For example, ability to modulate expression of a protein can be evaluated at the gene or protein level, e.g., using quantitative PCR or immunoassay methods. In some embodiments, high throughput methods, e.g., protein or gene chips as are known in the art (see, e.g., Ch. 12, Genomics, in Griffiths et al., Eds. Modern genetic Analysis, 1999,W. H. Freeman and Company; Ekins and Chu, Trends in Biotechnology, 1999, 17:217-218; MacBeath and Schreiber, Science 2000, 289(5485):1760-1763; Simpson, Proteins and Proteomics: A Laboratory Manual, Cold Spring Harbor Laboratory Press; 2002; Hardiman, Microarrays Methods and Applications: Nuts & Bolts, DNA Press, 2003), can be used to detect an effect on two, three, four, five or more of the proteins listed in Table 2, and/or in Fig. 4. Ability to modulate signaling via the kallikrein/kinin pathway can be evaluated, e.g., using liberation of bradykinin or other proteolytic products of kininogen (see, e.g., Campbell et al., Braz J Med Biol Res. 2000 Jun;33(6):665-77), and using the measurement of cyclic guanine monophosphate (cGMP). Vascular permeability can be evaluated, e.g., as described herein.

A test compound that has been screened by a method described herein and determined to inhibit PARP-1, can be considered a candidate compound. A candidate compound that has been screened, e.g., in an *in vivo* model of SPMS and determined to have a desirable effect on the disorder, e.g., on one or more symptoms of the disorder, can be considered a candidate therapeutic agent. Candidate therapeutic agents, once screened in a clinical setting, are therapeutic agents. Candidate compounds, candidate therapeutic agents, and therapeutic agents can be optionally optimized and/or derivatized, and formulated with physiologically acceptable excipients to form pharmaceutical compositions.

Thus, test compounds identified as "hits" (e.g., test compounds that inhibit PARP-1) in a first screen can be selected and systematically altered, e.g., using rational design, to optimize binding affinity, avidity, specificity, or other parameter. Such optimization can also be screened for using the methods described herein. Thus, in one embodiment, the disclosure includes screening a first library of compounds using a method known in the art and/or described herein, identifying one or more hits in that library, subjecting those hits to systematic structural alteration to create a second library of compounds structurally related to the hit, and screening the second library using the methods described herein.

Test compounds identified as hits can be considered candidate therapeutic compounds, useful in treating SPMS. A variety of techniques useful for determining the structures of "hits" can be used in the methods described herein, e.g., NMR, mass spectrometry, gas chromatography equipped with electron capture detectors, fluorescence and absorption spectroscopy. Thus, the disclosure comprises compounds identified as "hits" by the methods described herein, and methods for their administration and use in the treatment, prevention, or delay of development or progression of a disorder described herein.

Test compounds identified as candidate therapeutic compounds can be further screened by administration to an animal model of SPMS, as described herein. The animal can be monitored for a change in the disorder, e.g., for an improvement in a parameter of the disorder, e.g., a parameter related to clinical outcome. In some embodiments, the model is EAE and the parameter is a clinical signs of EAE, e.g., assessed according to the following score: 0, no signs of disease; 1, loss of tone in the tail; 2, hind limb paresis; 3, hind limb paralysis; 4, tetraplegia; 5, moribund, and an improvement would be a decrease in the EAE score, e.g., from 4 to 3. In some embodiments, the subject is a human, e.g., a human with SPMS, and the parameter is a clinical symptom of SPMS, and an improvement would result in a reduction of disability.

### EXAMPLES

The invention is further described in the following examples, which do not limit the scope of the invention described in the claims.

### Example 1: Increased 15-HC Serum Levels Are Linked to Human and Experimental SPMS

To investigate the role of 15-oxysterols in MS, we measured the levels of 15-ketocholestene (15-KE), 15-ketocholestane (15-KA) and 15-hydroxycholestene (15-HC) in sera from RRMS patients, SPMS patients and healthy controls (HC).

The serum samples from RRMS patients, SPMS patients, and controls were collected at the MS center of Brigham and Women's Hospital and at the University Hospital, School of Medicine, University of Sevilla. The clinical characteristics of the patients and controls are listed in Table 1.

**Table 1: Human patient samples analyzed**

| **Group** | **N** | **Gender (F/M)** | **Age** | **Disease Duration** | **EDSS** |
|---|---|---|---|---|---|
| **RRMS** | 12 | (9/3) | 37 (24-55) | 1.4 (0-4) | 1.6 (0-3.5) |
| **PPMS** | 9 | (5/4) | 56 (46-77) | 18.1 (3-25) | 5.3 (2-8) |
| **HC** | 8 | (6/2) | 34 (22-50) | NA | NA |

The N, gender, age, disease duration and EDSS for each group are indicated as the median and range. NA, not applicable.

15-oxysterols in the serum and CSF of the patients were analyzed by gas chromatography/mass spectrometry (GC/MS) as described (Diestel et al., J Exp Med 198, 1729-1740 (2003)). In brief, following lipid extraction, the samples were analyzed on Waters Quattro micro GC/MS/MS (Waters, Mildford, MA, USA). Peaks of interest were identified based on their relative retention time and ion mass spectrometry in comparison with external standards, and quantified by integration in relation to a standard curve.

There were increased levels of 15-KE and 15-KA in RRMS samples (P<0.05, Fig 1A), and a further up-regulation in SPMS (P<0.05, Fig 1A). Strikingly, 15-HC levels were only increased in serum samples from SPMS patients (*P*<0.01, Fig 1A).

Ten-week old non-obese diabetic (NOD) mice immunized with MOG₃₅₋₅₅ develop a form of EAE that resembles SPMS (Basso et al., J Clin Invest 118, 1532-1543 (2008)). In this model, a self-limited acute neurological syndrome peaks at day 18 after immunization and is followed by a partial recovery, after this initial attack follows a phase of irreversible progressive neurological impairment (Basso et al., J Clin Invest 118, 1532-1543 (2008)). This experimental model of SPMS was used to analyze the levels of 15-oxysterols at peak of the acute EAE attack (day 18) and during the progressive phase of EAE (day 55).

Ten week old female NOD mice (Jackson Laboratories, Bar Harbor, Maine, USA) were immunized with 100 µg of MOG₃₅₋₅₅ emulsified in CFA (Difco Laboratories) as described (Basso et al., J Clin Invest 118, 1532-1543 (2008)). 150 ng/mouse of pertussis toxin (Sigma) were administered intraperitoneally on days 0 and 2. Clinical signs of EAE were assessed according to the following score: 0, no signs of disease; 1, loss of tone in the tail; 2, hind limb paresis; 3, hind limb paralysis; 4, tetraplegia; 5, moribund.

The results indicated that 15-KA and 15-KE were up-regulated both during the acute attack and the progressive phase of EAE (Fig. 1B). 15-HC levels, however, were only up-regulated in the progressive phase of EAE (P<0.01, Fig. 1B), suggesting that 15-HC is linked to progressive neurological impairment both in human and experimental progressive MS.

Thus, serum levels of the 15-oxysterols 15-KA and 15-KE are up regulated both in RRMS and SPMS, with higher levels in SPMS. The serum and CSF levels of the oxysterol 7-KC have also been found increased in human and experimental MS (Diestel et al., J Exp Med 198, 1729-1740 (2003); Leoni et al., J Lipid Res 46, 191-195 (2005)), but their values fall within the normal range in the chronic phase of EAE (Diestel et al., J Exp Med 198, 1729-1740 (2003)). On the contrary, 15-HC serum levels are only increased in the progressive phase of human and experimental SPMS, suggesting that stage-specific processes control the production of 15-HC. The transition to SPMS has been linked to extensive microglia activation (Kutzelnigg et al. Brain 128, 2705-2712 (2005)). Since the degradation of myelin by microglia is an important source of oxysterols (Diestel et al., J Exp Med 198, 1729-1740 (2003); Bongarzone et al., J Neurosci Res 41, 213-221 (1995)), it may be (without wishing to be bound by theory) that changes in the CNS innate immune response alter the local catabolism of cholesterol and favor the generation of 15-HC in SPMS. Regardless of the specific mechanisms leading to higher 15-HC serum levels in SPMS, its association with human and experimental SPMS, and its contribution to disease pathogenesis by a TLR2/PARP-1 pathway suggest that 15-HC is a potential biomarker for monitoring disease progression. Further evidence for the use of 15-HC as a biomarker comes from its detection in serum samples, which facilitates its evaluation on large independent sets of samples and its use in the clinical set up.

### Example 2: PARP-1 Inhibition Suppresses Axonal Loss and Progressive Disability in Experimental SPMS

7-ketocholesterol has been reported to activate the nuclear enzyme PARP-1 (Diestel et al., J Exp Med 198, 1729-1740 (2003)). To investigate the role of PARP-1 in progressive MS we used the specific PARP-1 inhibitor 5-aminoisoquinolinone (AIQ) (Suto et al., Anticancer Drug Des 6, 107-117 (1991)) in the NOD experimental model of SPMS. AIQ administration had little or no effect on the acute phase of EAE, but it resulted in a significant inhibition of the clinical signs of progressive EAE (Figs. 2A-B).

In the NOD experimental model of SPMS, the progressive accumulation of clinical disability has been associated with extensive axonal loss and demyelination (Basso et al., J Clin Invest 118, 1532-1543 (2008)). The histological analysis of control and AIQ-treated mice showed that inhibition of PARP-1 with AIQ significantly reduced the axonal loss and demyelination linked to the progressive phase of NOD EAE (Fig. 2C). Thus, PARP-1 activation controls the progressive phase of experimental SPMS.

The suppression of the progressive phase of NOD EAE by AIQ was linked to the inhibition of PARP-1 activity in CNS CD11b⁺ cells but it did not affect the adaptive encephalitogenic immune response suggesting that PARP-1 contributes to the progression of MS by acting on the innate immune system at the CNS. This interpretation is in agreement with the activation of microglia observed in human and experimental SPMS (Basso et al., J Clin Invest 118, 1532-1543 (2008); Kutzelnigg et al. Brain 128, 2705-2712 (2005)) and the participation of PARP-1 in the damage of neurons by activated microglia in culture (Diestel et al., J Exp Med 198, 1729-1740 (2003) (Diestel et al., J Exp Med 198, 1729-1740 (2003); Kauppinen and Swanson, J Immunol 174, 2288-2296 (2005); Ullrich et al., Nat Cell Biol 3, 1035-1042 (2001)).

### Example 3: PARP-1 is Activated During Human and Experimental SPMS

To investigate the control of progressive EAE by PARP-1 the adaptive encephalitogenic response was studied in AIQ-treated mice.

Splenocytes and lymph node cells were cultured in X-VIVO medium and plated at 5 × 10⁵ cells per well in the presence of MOG₃₅₋₅₅ for 72 hours. During the last 16 hours, cells were pulsed with 1 µCi of [³H]-thymidine (PerkinElmer) followed by harvesting on glass fiber filters and analysis of incorporated [³H]-thymidine in a beta-counter (1450 Microbeta, Trilux; PerkinElmer). Supernatants were collect after 48 h of culture for cytokine measurements by ELISA.

Splenocytes from AIQ and control-treated mice showed similar proliferative responses upon activation with MOG₃₅₋₅₅ or mitogenic antibodies to CD3 (Fig. 3A), furthermore no differences were found on their profile of cytokine secretion (Fig. 7). Thus, the suppression of the progressive phase of experimental SPMS by AIQ does not result from its activity on the adaptive encephalitogenic response.

Microglia and astrocyte activation have been linked to the progressive phase of experimental SPMS (Basso et al., J Clin Invest 118, 1532-1543 (2008)), thus the activation of PARP-1 was analyzed in the CNS of NOD mice during the progressive phase of EAE. Antibodies to PAR, the product of PARP-1 activation, were used to detect active PARP-1 in CNS sections, in combination with antibodies to Iba-1 and GFAP, to identify microglia/CNS-infiltrating macrophages and astrocytes, respectively.

Spinal cord tissue samples from MOG-immunized NOD mice with or without AIQ treatment were collected at day 55, fixed in 4% paraformaldehyde, cryoprotected in 20% sucrose, and frozen in OCT. Spinal cord frozen sections were cut at 10 µm and processed for histological analysis, immunohistochemistry, or immunofluorescence. Axonal loss and demyelination were assessed by Bielschowsky's silver staining and Luxol Fast Blue, respectively (Basso et al., J Clin Invest 118, 1532-1543 (2008)). For image analysis, 3 or 4 lumbar spinal cord sections were selected according to regional landmarks from 4 different animals per group. Image analysis was performed using the software ImageJ (NIH). For quantification, level of labeling "threshold" was used as a cut-off to determine positive or negative staining. Immunofluorescence was performed using anti-Iba-1 (Wako, VA, USA), anti-GFAP (BD Bioscience, MA, USA) and anti PAR (Trevigen, MD, USA)

PARP-1 was activated in microglia/CNS-infiltrating macrophages and astrocytes during the progressive phase of NOD EAE (Fig 3B); PARP-1 activation was decreased in AIQ treated mice (data not shown). Similar results were obtained when PARP-1 enzymatic activity was measured in CNS CD11b⁺ cells isolated from NOD mice during the progressive phase of EAE (Fig. 3C).

To evaluate the relevance of these findings for human MS, PARP-1 activity was measured in MS patients. PARP-1 activity was measured using PARP HT Universal Colorimetric Kit (Trevigen, MD, USA), following manufacturer instructions. The activity of PARP-1 was increased in the peripheral blood monocytes of RRMS and SPMS patients (P<0.05 and *P*<0.01, Fig. 3D), with significantly higher levels in SPMS patients (P<0.05, Fig. 3D). Hence, these data suggests that PARP-1 activation is linked to human and experimental SPMS.

### Example 4: 15-HC Activates Microglia, Macrophages and Astrocytes via PARP-1

The oxidized derivative of cholesterol 7-KC has been shown to damage DNA and activate microglia by PARP-1 dependent pathway (Diestel et al., J Exp Med 198, 1729-1740 (2003)). Thus, the effect of 15-HC on PARP-1 activity was investigated using an in vitro system. The incubation of recombinant PARP-1 with either undamaged DNA or 15-HC alone did not have a significant effect on PARP-1 activity. However, the pre-incubation of DNA with 15-HC resulted in the activation of PARP-1 activation to levels comparable to those achieved with 7-KC or single strand break DNA (Fig. 5A).

The effect of 15-HC on the activity of cellular PARP-1 was then investigated. Incubation of macrophages, microglia or astrocytes with 15-HC resulted in a dose-dependent activation of PARP-1, similar to that achieved with 7-KC (Fig. 4B). The activation of PARP-1 by 15-HC could be inhibited with AIQ in a dose dependent manner (Fig. 4C). The incubation of microglia, macrophages or astrocytes with 15-HC resulted in the secretion of NO, CCL-2 and TNF-α, which could be inhibited by co-incubation with the PARP-1 specific inhibitor AIQ (Fig. 4D). Thus, 15-HC activates microglia, macrophages and astrocytes through a PARP-1-dependent mechanism.

Astrocytes, microglia and CNS-infiltrating monocytes influence the course of MS through several mechanisms that include the direct destruction of neurons by cytokines (Jack et al., J Neurosci Res 81, 363-373 (2005); Nair et al., Cell Mol Life Sci 65, 2702-2720 (2008)) or NO (Smith and Lassmann, Lancet Neurol 1, 232-241 (2002)), the recruitment of peripheral cells to the CNS (Charo and Ransohoff, N Engl J Med 354, 610-621 (2006)) and the secretion of cytokines that modulate peripheral adaptive immunity (Luo et al., Clin Invest 117, 3306-3315 (2007)). As described herein, the activation of microglia, macrophages and astrocytes with 15-HC in vitro resulted in the secretion of TNFa, NO and CCL2. Thus, without wishing to be bound by theory, the secretion of TNFa and NO triggered by 15-HC might be directly involved in axonal degeneration and disease progression (Jack et al., J Neurosci Res 81, 363-373 (2005); Nair et al., Cell Mol Life Sci 65, 2702-2720 (2008); Smith and Lassmann, Lancet Neurol 1, 232-241 (2002)), while the production of CCL2 could amplify this pathogenic mechanism by recruiting more pro-inflammatory cells to the CNS (Charo and Ransohoff, N Engl J Med 354, 610-621 (2006)).

### Example 5: 15-HC Activates PARP-1 by a TLR2-Dependent Pathway

Kinetic studies showed that the incubation of macrophages or microglia with 15-HC for 5 minutes results in the activation of PARP-1, while longer incubation times were required for 7-KC (Fig. 5A). These data suggested that 15-HC triggers additional pathways leading to PARP-1 activation. Reverse protein arrays (RPA) (Chan et al., Nat Med 10, 1390-1396 (2004)) were used to characterize the signaling pathways involved in the activation of PARP-1 by 15-HC. RPA were constructed by spotting microarrays of cell lysates prepared at different time points (0 to 30 minutes) after the activation of microglia or macrophages with 15-HC. The RPA were then probed with antibodies specific for proteins or phosphoproteins of linked to signaling pathways of interest (listed in Table 2).

The C8B4 microglial cell line, C8-D1A astrocytes and Raw 264.7 macrophages were purchased from ATCC (Manassas, VA, USA). Cells were cultured with DMEM supplemented with 10% fetal bovine serum, 2 mM L-glutamine, 25 mM HEPES, and 1 mM sodium pyruvate. Cells in logarithmic growth were treated with 1 µg/ml of 15-HC for 1, 5, 15, and 30 minutes in serum-free medium. Blocking antibodies (Ebioscience, San Diego, CA, USA) were added in some experiments at a final concentration of 10ug/ml. At indicated time points, the cells were lysed in an equal volume of 2X lysis buffer and snap-frozen to prevent any changes in phosphorylation. The cell lysates were transferred into 384-well polypropylene plates and spotted onto Super Epoxi slides (Telechem, Sunnyvale, CA, USA) using a robotic microarrayer (Genetix, Boston, MA, USA) fitted with solid spotting pins (Quintana et al., Proc Natl Acad Sci U S A 101 Suppl 2, 14615-14621 (2004)). Slides were then probed, processed and analyzed as previously described (Chan et al., Nat Med 10, 1390-1396 (2004)). Table 2 lists the phospho-specific antibodies used in our experiments.

**Table 2: Antibodies used for RPA analysis**

| | **Antibody** | **Source** |
|---|---|---|
| 1 | Phospho-E1k-1 (Ser383) Antibody | Cell Signaling Technologies |
| 2 | Phospho-p38 MAPK (Thr180/Tyr182) Antibody | Cell Signaling Technologies |
| 3 | Phospho-p38 MAPK (Thr180/Tyr182) Antibody | Abcam |
| 4 | Phospho-p38 MAP Kinase (Thr180/Tyr182) Antibody | Cell Signaling Technologies |
| 5 | Phospho-MKK3/MKK6 (Ser189/207) Antibody | Cell Signaling Technologies |
| 6 | Phospho-ATF-2 (Thr71) Antibody | Cell Signaling Technologies |
| 7 | Phospho-HSP27 (Ser82) Antibody | Cell Signaling Technologies |
| 8 | Phospho-MAPKAPK-2 (Thr334) Antibody | Cell Signaling Technologies |
| 9 | Phospho-SEK1/MKK4 (Thr261) Antibody | Cell Signaling Technologies |
| 10 | Phospho-SAPK/JNK (Thr183/Tyr185) Antibody | Cell Signaling Technologies |
| 11 | Phospho-c-Jun (Ser63) II Antibody | Cell Signaling Technologies |
| 12 | Phospho-Akt (Ser473) Antibody | Cell Signaling Technologies |
| 13 | Phospho-Akt (Thr308) Antibody | Cell Signaling Technologies |
| 14 | Akt Antibody Antibody | Cell Signaling Technologies |
| 15 | Phospho-GSK-3beta (Ser9) Antibody | Cell Signaling Technologies |
| 16 | Phospho-Raf(Ser259) Antibody | Cell Signaling Technologies |
| 17 | Phospho-PTEN (Ser380) Antibody | Cell Signaling Technologies |
| 18 | Phospho-PDK1 (Ser241) Antibody | Cell Signaling Technologies |
| 19 | Phospho-Stat1 (Tyr701) Antibody | Cell Signaling Technologies |
| 20 | Phospho-Stat2 (Tyr690) Antibody | Cell Signaling Technologies |
| 21 | Phospho-Stat3 (Tyr705) Antibody | Cell Signaling Technologies |
| 22 | Phospho-Stat3 (Ser727) Antibody | Cell Signaling Technologies |
| 23 | Phospho-Stat5 (Tyr694) Antibody | Cell Signaling Technologies |
| 24 | Phospho-Stat6 (Tyr641) Antibody | Cell Signaling Technologies |
| 25 | Phospho-c-Raf (Ser338) (56A6) Antibody | Cell Signaling Technologies |
| 26 | Phospho-Src Family (Tyr416) Antibody | Cell Signaling Technologies |
| 27 | Phospho-Pyk2 (Tyr402) Antibody | Cell Signaling Technologies |
| 28 | Pyk2 Antibody | Cell Signaling Technologies |
| 29 | Phospho-MEK1/2 (Ser217/221) Antibody | Cell Signaling Technologies |
| 30 | Phospho-MEK1/2 (Ser221) Antibody | Cell Signaling Technologies |
| 31 | Phospho-p44/42 MAPK (Thr202/Tyr204) Antibody | Cell Signaling Technologies |
| 32 | Phospho-p44/42 MAP Kinase (Thr202/Tyr204) Antibody | Cell Signaling Technologies |
| 33 | Phospho-Erk 1/2 (Thr202/Tyr204) Antibody | Cell Signaling Technologies |
| 34 | Phospho-p90RSK (Ser380) Antibody | Cell Signaling Technologies |
| 35 | Phospho-PKC (pan) (BetaII Ser660) Antibody | Cell Signaling Technologies |
| 36 | Phospho-PKCalpha/betaII (Thr638/641) Antibody | Cell Signaling Technologies |
| 37 | Phospho-PKCdelta (Thr505) Antibody | Cell Signaling Technologies |
| 38 | Phospho-PKCdelta (Ser643) Antibody | Cell Signaling Technologies |
| 39 | Phospho-PKD/PKCmu (Ser744/748) Antibody | Cell Signaling Technologies |
| 40 | Phospho-PKD/PKCmu (Ser916) Antibody | Cell Signaling Technologies |
| 41 | PKD/PKCmu Antibody | Cell Signaling Technologies |
| 42 | Phospho-PKCtheta (Thr538) Antibody | Cell Signaling Technologies |
| 43 | Phospho-PKCzeta/lambda (Thr410/403) Antibody | Cell Signaling Technologies |
| 44 | Phospho-PLCgamma2 (Tyr1217) Antibody | Cell Signaling Technologies |
| 45 | Phospho-PLCgamma1 (Tyr771) Antibody | Cell Signaling Technologies |
| 46 | Phospho-NFKB2 p100 (Ser866/870) Antibody | Cell Signaling Technologies |
| 47 | Phospho-NF-kappaB p105 (Ser933) Antibody | Cell Signaling Technologies |
| 48 | Phospho-NF-κB p65 (Ser536) Antibody | Cell Signaling Technologies |
| 49 | Phospho-IKKalpha/beta (Ser176/180) Antibody II | Cell Signaling Technologies |
| 50 | Phospho-IkappaB-alpha (Ser32) Antibody | Cell Signaling Technologies |
| 51 | Phospho-Akt Substrate (RXRXXS/T) (110B7) Antibody | Cell Signaling Technologies |
| 52 | Phospho-(Ser) PKC Substrate Antibody | Cell Signaling Technologies |
| 53 | Phospho-PKA Substrate (RRXS/T) (100G7) Antibody | Cell Signaling Technologies |
| 54 | Phospho-SHIP1 Antibody | Cell Signaling Technologies |
| 55 | Phospho-(Ser) CDKs Substrate Antibody | Cell Signaling Technologies |
| 56 | Phospho-(Ser/Thr) ATM/ATR Substrate Antibody | Cell Signaling Technologies |
| 57 | Phospho-IRAK1 (Ser376) Antibody | Cell Signaling Technologies |
| 58 | Phospho-TAK1 (Thr184/187) (90C7) Antibody | Cell Signaling Technologies |
| 59 | Phospho-IRF-3 (Ser396) Antibody | Cell Signaling Technologies |
| 60 | Phospho-IRAK (Thr209) Antibody | Abcam |
| 61 | Phospho-MKK7(Ser271/Thr275) Antibody | Cell Signaling Technologies |
| 62 | Phospho-Tp12 (Ser 400) Antibody | Cell Signaling Technologies |
| 63 | Phospho-PI3K p85 (Tyr458)/p55 (Tyr199) Antibody | Cell Signaling Technologies |
| 64 | Phospho-MARCKS (Ser 152-156) Antibody | Prosci |
| 65 | Phospho-cFos (Thr232) Antibody | Prosci |
| 66 | Tp12 Antibody | Cell Signaling Technologies |
| 67 | PI3K p85 Antibody | Cell Signaling Technologies |

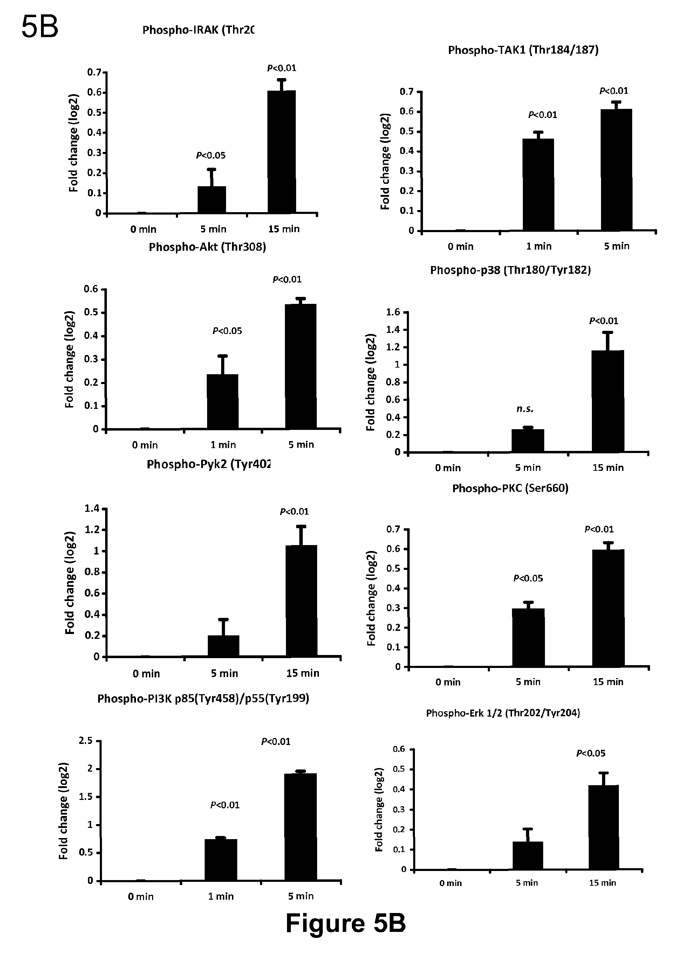

The treatment of microglia or macrophage cells by 15-HC resulted in the phosphorylation of proteins that mediate TLR signaling, including TAK1, IRAK, Akt, p38-MAPK, PyK2, PKC, PI3K and ERK1/2 (Akira et al., Cell. 124, 783-801 (2006)) (Fig. 5B); similar results were obtained when these proteins were analyzed by western blot (Fig. 8). Specific inhibitors were then used to investigate the functional relevance of the protein kinases identified with RPA for the activation of PARP-1 by 15-HC. Treatment of microglia or macrophages with 15-HC in the presence of specific inhibitors of Erk, Akt, PI3K, PKC or p38-MAPK resulted in a significant inhibition of PARP-1 activation (Fig. 5C).

Erkl/2 kinases have been shown to physically interact with PARP-1, resulting in PARP-1 activation by a mechanism independent of DNA damage (Cohen-Armon et al., Mol Cell 25, 297-308 (2007)). Thus, co-immunoprecipitation studies were carried out to better characterize the mechanisms by which 15-HC activates PARP-1. Microglia and macrophages were activated with 15-HC, ERK-specific antibodies were used for immunoprecipitation, and the pulled down material was probed with antibodies to PARP-1. Treatment of microglia and macrophages with 15-HC resulted in a significant interaction of Erk with PARP-1 (Fig. 5D).

Blocking antibodies were then used to confirm the role of TLR in the activation of PARP-1 by 15-HC, and to identify the specific TLR involved. Antibodies to TLR2, but not to TLR4 inhibited the activation of PARP-1 by 15-HC; no effect was seen when isotype matched control antibodies were used (Fig. 6A). This inhibition of the activation of PARP-1 by 15-HC correlated with a complete suppression of the phosphorylation of TAK1, IRAK, Akt, p38-MAPK, PyK2, PKC, PI3K and ERK1/2 (Fig. 9).

To further confirm the role of TLR2 in PARP-1 activation by 15α-HC the human embryonic kidney cell line HEK-293, which expresses PARP-1 but does not express TLR2. was used. Treatment of HEK-293 cells with 15-HC had no significant effect in PARP-1 activity (Fig. 6B). However, transfection of HEK-293 cells with a plasmid coding for TLR2 resulted in a significant activation of PARP-1 by 15α-HC (Fig. 6B). Taken together these results suggest that 15-HC activates microglia, macrophages and astrocytes trough a TLR2/PARP-1-dependent signaling pathway.

Considering the higher serum levels of 15-HC and the increased activation of monocyte PARP-1 that we found in human SPMS, 15-HC acting through a TLR2/PARP-1 signaling pathway is likely to contribute to the activation of microglia that characterizes SPMS (Kutzelnigg et al. Brain 128, 2705-2712 (2005)). These observations are in agreement with a model where dysregulated CNS innate immunity promotes neurodegeneration and the accumulation of disability in SPMS. Accordingly, new therapeutic approaches for SPMS should target both specific mechanisms of neurodegeneration like glutamate toxicity (Basso et al., J Clin Invest 118, 1532-1543 (2008); Pitt et al., Nat Med 6, 67-70 (2000); Smith et al., Nat Med 6, 62-66 (2000)) and the innate immune response that drives them. As demonstrated herein, PARP-1 is a potential therapeutic target to halt the CNS immune response that promotes axonal loss and the accumulation of disability in human and experimental model of SPMS.

All in all, our data suggest that 15-HC, acting via a TLR2/PARP-1-dependent signaling pathway, perpetuates local innate immune inflammation in the CNS during SPMS. Myelin degradation results in the production of 15-HC, which activates CNS innate immunity, leading to additional axonal destruction and the production of more 15-HC. Thus, the TLR2/ PARP-1 axis is a potential therapeutic target for SPMS and 15-HC might constitute a new biomarker for monitoring disease progression in MS.

### Example 6: The TLR2/PARP-1 Axis in SPMS

To investigate whether the TLR2/PARP-1 axis is functional in human we activated PBMCs from healthy controls with 15-HC, in the presence or not of PARP-1 or TLR2 inhibitors. Fig. 10A shows that treatment of human PBMCs with 1 ug/ml 15-HC results in the activation of PARP-1 enzymatic activity as measured using an enzymatic activity detection kit (i.e., the PARP HT Universal Colorimetric Kit (Trevigen)). This activation can be inhibited with the PARP-1 inhibitor AIQ or a TLR2 blocking antibody, but not by an isotype control antibody (Fig. 10A). Similarly to what was observed in mice, the activation of human monocytes by lug/ml 15-HC resulted in production of TNFa and CCL2 in a PARP-1 and TLR2 dependent manner (Fig. 10B). Thus, 15-HC activates PARP-1 in human monocytes by a TLR2 dependent pathway that results in the release of the pro-inflammatory mediators TNFa and CCL2.

A new FACS-based method was developed to detect PARP-1 activation based on the intracellular detection of the PARP-1 product PAR with specific antibodies. Briefly, the cells were incubated with antibodies to CD 16/32 to block unspecific antibody binding, and then stained for surface markers. After washing, the cells were permeabilized with Fix/Perm (BD), and then stained with rabbit anti-PAR antibodies (or control rabbit IgG) for 30' at 4°C, followed by anti-rabbit PE for 30' at room temperature. The samples were then analyzed by FACS.

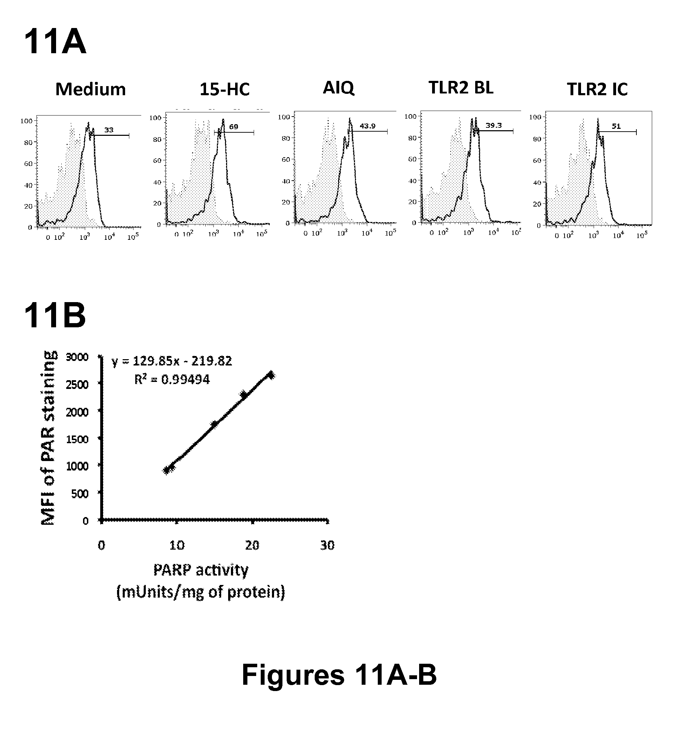

Activation of human monocytes by 15-HC resulted in a significant increase in PAR staining (Fig. 11A), which could be blocked with the specific PARP-1 inhibitor AIQ or with blocking antibodies to TLR2 (Fig. 11A). There was a linear correlation between the PARP-1 activity detected on human monocytes using the enzymatic kit or the newly-developed FACS based method (Fig. 11B).

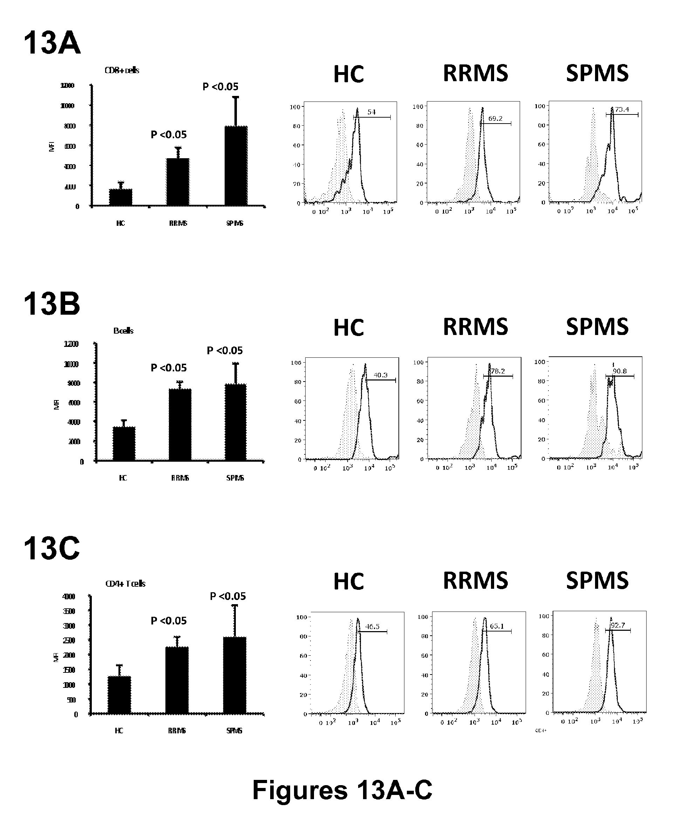

The FACS-based method was used to characterize the activity of PARP-1 in cells of the innate immune system present in the circulation of RRMS, SPMS patients and healthy controls. There was a significant up-regulation of PARP-1 activity in the inflammatory monocytes (Fig. 12A), classical monocytes (Fig. 12B) and DC (Fig. 12C) of SPMS patients. This up-regulation was significant when compared to the PARP-1 activity detected on healthy controls (HC) or RRMS patients. When cells of the adaptive immune system of the same patients were analyzed, PARP-1 activity was elevated both in RRMS and SPMS patients in CD8+ T cells (Fig. 13A), B cells (Fig. 13B) and CD4+ CD25- T cells (Fig. 13C). Thus, PARP-1 activity is up-regulated in SPMS patients in cells of the innate immune system, and in both RRMS and SPMS patients in cells of the adaptive immune system.

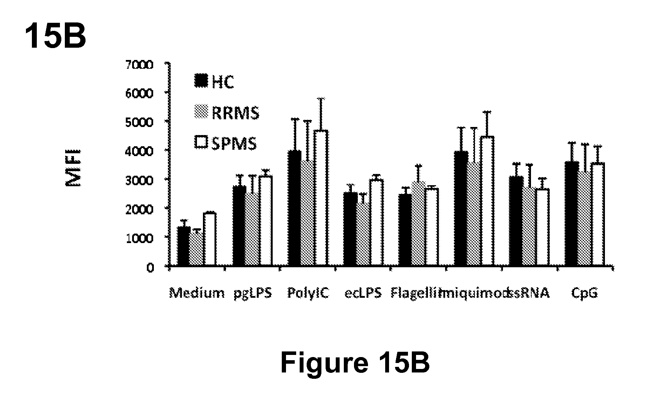

PARP-1 activity in SPMS and RRMS was then evaluated in monocytes to determine if the differences between SPMS and RRMS resulted from an exacerbated response to 15-HC or other TLR agonists in the monocytes from SPMS patients. Upon incubation with 15-HC, monocytes from healthy controls, RRMS and SPMS patients reached similar levels of PARP-1 activation (Figs. 14A and 14B). Moreover, in these three experimental groups, the activation of PARP-1 by 15-HC could be inhibited with the PARP-1 specific inhibitor 15-HC and TLR2 blocking antibodies (Figs. 14A and 14B). Moreover, there were no significant differences in the response to other TLR agonists that are also known to trigger the activation of PARP-1 (Figs. 15A and 15B). Thus, the increased PARP-1 activity found in the innate immune system from SPMS patients does not result from a hyper-reactive TLR/PARP-1 signaling axis.

To investigate whether the increased activity of the TLR2/PARP-1 axis in SPMS patients resulted from increased levels of TLR2 ligands, the levels of 15-HC and HSP60 were measured in serum samples from healthy controls, RRMS and SPMS patients. There were increased levels of HSP60 in serum samples of RRMS patients (Fig. 16A) and increased levels of 15-HC (Fig. 16B) in serum samples of SPMS patients. Since both HSP60 and 15-HC have been reported to be TLR2 agonists, their ability to activate cells of the innate and the adaptive immune system was compared in vitro. Different cells of the adaptive or innate immune system were incubated with different concentrations of HSP60 (1, 5, or 10 ng/ml) or 15-HC (1, 5, or 10 ug/ml). The results indicated that only 15-HC could activate cells of the adaptive immune system (Fig. 17), although both HSP60 and 15-HC could activate cells of the adaptive immune response (Fig. 18). Thus, although both HSP60 and 15-HC are TLR2 ligands, they differ in their ability to activate the TLR2/PARP-1 axis in cells of the adaptive or the innate immune system. Moreover, these data indicates that increased levels of 15-HC drive the innate immunity activation via PARP-1 in the progressive stage of MS.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method of diagnosing multiple sclerosis in a human subject, the method comprising:
evaluating a level of one or more 15-oxysterol in a serum sample from the human subject by a method comprising subjecting the serum sample to lipid extraction and analysing the sample using tandem quadrupole GC-MS-MS; and
comparing the level of the 15-oxysterol in the sample with a reference level,
wherein the level of the 15-oxysterol in the sample in comparison to the reference is indicative of whether the human subject has multiple sclerosis.

2. The method of claim 1, wherein the one or more 15-oxysterol is selected from the group consisting of 15-ketocholestene (15-KE), 15-ketocholestane (15-KA), and 15-hydroxycholestene (15-HC).

3. The method of claim 1, wherein the reference level is a control reference that represents a normal level of the 15-oxysterol.

4. The method of claim 3, wherein the normal level is a level in an unaffected human subject.

5. The method of claim 1, wherein the reference level is a disease reference that represents a level of the 15-oxysterols in a reference human subject having multiple sclerosis.

6. The method of claim 5, wherein the reference human subject has relapsing-remitting multiple sclerosis (RRMS) or secondary progressive multiple sclerosis (SPMS).

7. A method of determining whether a human subject has relapsing-remitting multiple sclerosis-(RRMS) or secondary progressive multiple sclerosis (SPMS), the method comprising:
evaluating a level of one or both of 15-ketocholestene (15-KE) or 15-ketocholestane (15-KA), and also evaluating the presence and/or level of 15-hydroxycholestene (15-HC), in a serum sample from the human subject by a method comprising subjecting the serum sample to lipid extraction and analysing the sample using tandem quadrupole GC-MS-MS; and
comparing the level of 15-KE and/or 15-KA, and 15-HC in the sample with corresponding references,
wherein the level of 15-KE and/or 15-KA, and 15-HC in the sample as compared to the control reference indicates whether the human subject has RRMS or SPMS.

8. The method of claim 7, wherein the reference is a control reference represents a normal level of the 15-KE or 15-KA, and 15-HC in an unaffected human subject, or a disease reference that represents a level in a human subject having RRMS, or SPMS.

9. The method of claim 7, wherein the presence of elevated levels of 15-KE or 15-KA, and elevated levels of 15-HC as compared to a reference, indicates that the human subject has SPMS or has an increased risk of developing SPMS, while increased levels of 15-KE, and 15-KA, but not 15-HC, as compared to the reference indicates that the human subject has RRMS or has not yet progressed to SPMS.

10. A method of diagnosing SPMS in a human subject, the method comprising:
evaluating a level of 15-hydroxycholestene (15-HC) in a serum sample from the human subject by a method comprising subjecting the serum sample to lipid extraction and analysing the sample using tandem quadrupole GC-MS-MS; and
comparing the level of 15-HC in the sample with a corresponding reference,
wherein the level of 15-HC in the sample as compared to the reference is indicative of whether the human subject has SPMS.

11. The method of claim 10, wherein the reference is a control reference that represents a normal level of 15-HC in an unaffected human subject, and/or a disease reference that represents a level in a human subject having SPMS.

12. The method of claim 11, wherein the reference is a control reference that represents a normal level of 15-HC in an unaffected human subject, and the presence of elevated 15-HC indicates that the human subject has SPMS.

## Patentansprüche

1. Verfahren zur Diagnose von multipler Sklerose in einem menschlichen Probanden, wobei das Verfahren Folgendes umfasst:
Bewerten eines Spiegels von einem oder mehr 15-Oxysterolen in einer Serumprobe von dem menschlichen Probanden durch ein Verfahren, das umfasst, dass die Serumprobe der Lipidextraktion und Analyse der Probe mithilfe von Tandem-Quadrupol-GC-MS/MS unterzogen wird; und
Vergleichen des Spiegels des 15-Oxysterols in der Probe mit einem Bezugsspiegel,
worin der Spiegel des 15-Oxysterols in der Probe im Vergleich zum Bezug dafür indikativ ist, ob der menschliche Proband multiple Sklerose hat.

2. Verfahren nach Anspruch 1, worin das eine oder die mehreren 15-Oxysterole aus der Gruppe ausgewählt werden, die aus 15-Ketocholesten (15-KE), 15-Ketocholestan (15-KA) und 15-Hydroxycholesten (15-HC) besteht.

3. Verfahren nach Anspruch 1, worin der Bezugsspiegel ein Kontrollbezug ist, der einen normalen Spiegel des 15-Oxysterols darstellt.

4. Verfahren nach Anspruch 3, worin der normale Spiegel ein Spiegel in einem nicht betroffenen menschlichen Probanden ist.

5. Verfahren nach Anspruch 1, worin der Bezugsspiegel ein Krankheitsbezug ist, der einen Spiegel der 15-Oxysterole in einem menschlichen Bezugsprobanden mit multipler Sklerose darstellt.

6. Verfahren nach Anspruch 5, worin der menschliche Bezugsproband schubförmigwiederkehrende multiple Sklerose (RRMS) oder sekundär-progrediente multiple Sklerose (SPMS) hat.

7. Verfahren zum Bestimmen dessen, ob ein menschlicher Proband schubförmigwiederkehrende multiple Sklerose (RRMS) oder sekundär-progrediente multiple Sklerose (SPMS) hat, wobei das Verfahren Folgendes umfasst:
Bewerten eines Spiegels von einem oder beiden von 15-Ketocholesten (15-KE) oder 15-Ketocholestan (15-KA), sowie Bewerten der Präsenz und/oder des Spiegels von 15-Hydroxycholesten (15-HC), in einer Serumprobe von dem menschlichen Probanden durch ein Verfahren, das umfasst, dass die Serumprobe der Lipidextraktion und Analyse der Probe mithilfe von Tandem-Quadrupol-GC-MS/MS unterzogen wird; und
Vergleichen des Spiegels von 15-KE und/oder 15-KA und 15-HC in der Probe mit entsprechenden Bezügen,
worin der Spiegel von 15-KE und/oder 15-KA und 15-HC in der Probe im Vergleich zum Kontrollbezug angibt, ob der menschliche Proband RRMS oder SPMS hat.

8. Verfahren nach Anspruch 7, worin der Bezug ein Kontrollbezug ist, der einen normalen Spiegel des 15-KE oder 15-KA und 15-HC in einem nicht betroffenen menschlichen Probanden darstellt, oder ein Krankheitsbezug, der einen Spiegel in einem menschlichen Probanden mit RRMS oder SPMS darstellt.

9. Verfahren nach Anspruch 7, worin die Präsenz von erhöhten Spiegeln von 15-KE oder 15-KA und erhöhten Spiegeln von 15-HC im Vergleich zu einem Bezug angibt, dass der menschliche Proband SPMS hat oder bei ihm ein erhöhtes Risiko des Auftretens von SPMS besteht, während erhöhte Spiegel von 15-KE und 15-KA, aber nicht 15-HC, im Vergleich zu einem Bezug angeben, dass der menschliche Proband RRMS hat oder noch nicht in SPMS übergegangen ist.

10. Verfahren zur Diagnose von SPMS in einem menschlichen Probanden, wobei das Verfahren Folgendes umfasst:
Bewerten eines Spiegels von 15-Hydroxycholesten (15-HC) in einer Serumprobe von dem menschlichen Probanden durch ein Verfahren, das umfasst, dass die Serumprobe der Lipidextraktion und Analyse der Probe mithilfe von Tandem-Quadrupol-GC-MS/MS unterzogen wird; und
Vergleichen des Spiegels von 15-HC in der Probe mit einem entsprechenden Bezug,
worin der Spiegel von 15-HC in der Probe im Vergleich zum Bezug dafür indikativ ist, ob der menschliche Proband SPMS hat.

11. Verfahren nach Anspruch 10, worin der Bezug ein Kontrollbezug ist, der einen normalen Spiegel von 15-HC in einem nicht betroffenen menschlichen Probanden darstellt, und/oder ein Krankheitsbezug, der einen Spiegel in einem menschlichen Probanden mit SPMS darstellt.

12. Verfahren nach Anspruch 11, worin der Bezug ein Kontrollbezug ist, der einen normalen Spiegel von 15-HC in einem nicht betroffenen menschlichen Probanden darstellt, und die Präsenz von erhöhtem 15-HC angibt, dass der menschliche Proband SPMS hat.

## Revendications

1. Procédé de diagnostic d'une sclérose en plaques chez un sujet humain, le procédé consistant à :
évaluer un niveau d'un ou plusieurs 15-oxystérols dans un échantillon de sérum provenant du sujet humain par un procédé consistant à soumettre l'échantillon de sérum à une extraction de lipides et à analyser l'échantillon au moyen d'une technique GC-MS-MS quadripôle en tandem ; et
comparer le niveau du 15-oxystérol dans l'échantillon à un niveau de référence,
le niveau du 15-oxystérol dans l'échantillon comparé à la référence indiquant si le sujet humain souffre d'une sclérose en plaques.

2. Procédé selon la revendication 1, dans lequel le ou les 15-oxystérols sont choisis dans le groupe constitué de 15-cétocholestène (15-KE), 15-cétocholestane (15-KA) et 15-hydroxycholestène (15-HC).

3. Procédé selon la revendication 1, dans lequel le niveau de référence est une référence témoin qui représente un niveau normal de 15-oxystérol.

4. Procédé selon la revendication 3, dans lequel le niveau normal est un niveau présent chez un sujet humain non atteint.

5. Procédé selon la revendication 1, dans lequel le niveau de référence est une référence de maladie qui représente un niveau de 15-oxystérol chez un sujet humain de référence atteint d'une sclérose en plaques.

6. Procédé selon la revendication 5, dans lequel le sujet humain de référence est atteint d'une sclérose en plaques rémittente-récurrente (SEP RR) ou d'une sclérose en plaques secondaire progressive (SEP SP).

7. Procédé consistant à déterminer si un sujet humain est atteint d'une sclérose en plaques rémittente-récurrente (SEP RR) ou d'une sclérose en plaques secondaire progressive (SEP SP), le procédé consistant à :
évaluer un niveau de 15-cétocholestène (15-KE) et/ou de 15-cétocholestane (15-KA) et évaluer aussi la présence et/ou le niveau de 15-hydroxycholestène (15-HC) dans un échantillon de sérum provenant du sujet humain par un procédé consistant à soumettre l'échantillon de sérum à une extraction de lipides et à analyser l'échantillon au moyen d'une technique GC-MS-MS quadripôle en tandem ; et
comparer le niveau de 15-KE et/ou de 15-KA ainsi que de 15-HC dans l'échantillon à des références correspondantes,
le niveau de 15-KE et/ou de 15-KA ainsi que de 15-HC dans l'échantillon comparé à la référence témoin indiquant que le sujet humain souffre d'une SEP RR ou d'une SEP SP.

8. Procédé selon la revendication 7, dans lequel la référence est une référence témoin qui représente un niveau normal de 15-KE ou de 15-KA ainsi que de 15-HC chez un sujet humain non atteint, ou une référence de maladie qui représente un niveau chez un sujet humain atteint d'une SEP RR ou d'une SEP SP.

9. Procédé selon la revendication 7, dans lequel la présence de niveaux élevés de 15-KE ou de 15-KA ainsi que de niveaux élevés de 15-HC comparés à une référence, indique que le sujet humain souffre d'une SEP SP ou présente un risque accru de développer une SEP SP, tandis que des niveaux élevés de 15-KE et de 15-KA, mais pas de 15-HC, comparés à la référence, indiquent que le sujet humain souffre d'une SEP RR ou d'une SEP RR qui n'a pas encore évolué vers une SEP SP.

10. Procédé de diagnostic d'une SEP SP chez un sujet humain, le procédé consistant à :
évaluer un niveau de 15-hydroxycholestène (15-HC) dans un échantillon de sérum provenant du sujet humain par un procédé consistant à soumettre l'échantillon de sérum à une extraction de lipides et à analyser l'échantillon au moyen d'une technique GC-MS-MS quadripôle en tandem ; et
comparer le niveau de 15-HC dans l'échantillon à une référence correspondante,
le niveau de 15-HC dans l'échantillon comparé à la référence indiquant si le sujet humain souffre d'une SEP SP.

11. Procédé selon la revendication 10, dans lequel la référence est une référence témoin qui représente un niveau normal de 15-HC chez un sujet humain non atteint, et/ou une référence de maladie qui représente un niveau chez un sujet humain atteint d'une SEP SP.

12. Procédé selon la revendication 11, dans lequel la référence est une référence témoin qui représente un niveau normal de 15-HC chez un sujet humain non atteint, et dans lequel la présence d'un niveau élevé de 15-HC indique que le sujet humain souffre d'une SEP SP.
